(19) Europäisches Patentamt
European Patent Office
Office européen des brevets

(11) EP 2 429 600 B1

(12) **EUROPEAN PATENT SPECIFICATION**

(45) Date of publication and mention of the grant of the patent:
**06.09.2017 Bulletin 2017/36**

(21) Application number: **10775175.2**

(22) Date of filing: **17.05.2010**

(51) Int Cl.:
***A61L 27/44*** *(2006.01)*
***A61L 27/58*** *(2006.01)*
***C08L 89/00*** *(2006.01)*
***A61L 27/52*** *(2006.01)*
***C08L 5/00*** *(2006.01)*
***A61L 27/38*** *(2006.01)*
***C08L 5/04*** *(2006.01)*
***C08L 5/12*** *(2006.01)*
***C08L 89/06*** *(2006.01)*

(86) International application number:
**PCT/SG2010/000187**

(87) International publication number:
**WO 2010/132028 (18.11.2010 Gazette 2010/46)**

(54) **COMPOSITION FOR MANUFACTURING A SCAFFOLD FOR TISSUE ENGINEERING, AND A METHOD OF MAKING IT**

ZUSAMMENSETZUNG ZUR HERSTELLUNG EINES GERÜSTS ZUR GEWEBEZÜCHTUNG UND HERSTELLUNGSVERFAHREN DAFÜR

COMPOSITION ET PROCEDE DE FABRICATION D'UN ECHAFAUDAGE DE GENIE TISSULAIRE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **15.05.2009 US 178697 P**

(43) Date of publication of application:
**21.03.2012 Bulletin 2012/12**

(73) Proprietor: **Nanyang Technological University**
**Singapore 639798 (SG)**

(72) Inventors:
- **WANG, Dongan**
  **Singapore 639798 (SG)**
- **SU, Kai**
  **Singapore 639798 (SG)**
- **GONG, Yihong**
  **Singapore 639798 (SG)**
- **LAU, Ting Ting**
  **Singapore 639798 (SG)**

(74) Representative: **Viering, Jentschura & Partner mbB**
**Patent- und Rechtsanwälte**
**Kennedydamm 55 / Roßstrasse**
**40476 Düsseldorf (DE)**

(56) References cited:

**EP-A1- 2 011 816**
**WO-A2-2005/020849**
**US-A1- 2002 150 604**
**US-A1- 2008 206 308**
**US-A1- 2009 017 096**
**US-B1- 6 685 963**
**US-B2- 6 656 488**

- **SUH SOO WON ET AL: "Effect of different particles on cell proliferation in polymer scaffolds using a solvent-casting and particulate leaching technique", ASAIO JOURNAL, LIPPINCOTT WILLIAMS & WILKINS / ASAIO, HAGERSTOWN, MD, US, vol. 48, no. 5, 1 September 2002 (2002-09-01), pages 460-464, XP008111820, ISSN: 1058-2916**
- **LICHUN, L. ET AL.: 'Three-Dimensional Porous Biodegradable Polymeric Scaffolds Fabricated with Biodegradable Hydrogel Porogens' TISSUE ENGINEERING PART C: METHODS. vol. 15, no. 4, 2009, pages 583 - 594, XP055094305**
- **TABATA, Y. ET AL.: 'In Vivo Release of Plasmid DNA from Composites of Oligo(poly(ethylene glycol)fumerate) and Cationized Gelatin Microspheres' JOURNAL OF CONTROLLED RELEASE. vol. 107, no. 3, 2005, pages 547 - 561, XP027664123**
- **HENNINK, W., M. ET AL.: 'Degradation Behavior of Dextran Hydrogels Composed of Positively and Negatively Charged Microspheres' BIOMATERIALS vol. 27, 2006, pages 4141 - 4148, XP027951320**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Nanyang Technological University

<120> COMPOSITION FOR MANUFACTURING A SCAFFOLD FOR TISSUE ENGINEERING, AND A METHOD OF MAKING IT

<130> P47917

<140> 10 775 175.2
<141> 2010-05-17

<150> US 61/178,697
<151> 2009-05-15

<160> 20

<170> PatentIn version 3.5

<210> 1
<211> 20
<212> DNA
<213> Artificial Sequence

<220>
<223> Forward Primer Sequence for GAPDH

<400> 1
accccttcat tgacctccac 20

<210> 2
<211> 20
<212> DNA
<213> Artificial Sequence

<220>
<223> Reverse Primer Sequence For GAPDH

<400> 2
atactcagca ccagcatcgc 20

<210> 3
<211> 20
<212> DNA
<213> Artificial Sequence

<220>
<223> Forward Primer Sequence For HPRT1

<400> 3
ggacttgaat catgtttgtg 20

<210> 4
<211> 20
<212> DNA
<213> Artificial Sequence

<220>
<223> Reverse Primer Sequence For HPRT1

<400> 4
cagatgtttc caaactcaac 20

<210> 5
<211> 20
<212> DNA
<213> Artificial Sequence

<220>
<223> Forward Primer Sequence For RPL4

<400> 5
caagagtaac tacaaccttc 20

<210> 6
<211> 20
<212> DNA

<213> Artificial Sequence

<220>
<223> Reverse Primer Sequence For RPL4

<400> 6
gaactctacg atgaatcttc 20

<210> 7
<211> 25
<212> DNA
<213> Artificial Sequence

<220>
<223> Forward Primer Sequence For TBP1

<400> 7
aacagttcag tagttatgag ccaga 25

<210> 8
<211> 20
<212> DNA
<213> Artificial Sequence

<220>
<223> Reverse Primer Sequence For TBP1

<400> 8
agatgttctc aaacgcttcg 20

<210> 9
<211> 24
<212> DNA
<213> Artificial Sequence

<220>
<223> Forward Primer Sequence For Collagen II

<400> 9
gctatggaga tgacaacctg gctc 24

<210> 10
<211> 24
<212> DNA
<213> Artificial Sequence

<220>
<223> Reverse Primer Sequence For Collagen II

<400> 10
cacttaccgg tgtgtttcgt gcag 24

<210> 11
<211> 22
<212> DNA
<213> Artificial Sequence

<220>

<223> Forward Primer Sequence For Aggrecan

<400> 11
cgaggagcag gagtttgtca ac 22

<210> 12
<211> 22
<212> DNA
<213> Artificial Sequence

<220>
<223> Reverse Primer Sequence For Aggrecan

<400> 12
atcatcacca cgcagtcctc tc 22

<210> 13
<211> 20
<212> DNA
<213> Artificial Sequence

<220>
<223> Forward Primer Sequence For RhoA

<400> 13
agctgggcag gaagattatg 20

<210> 14
<211> 20
<212> DNA
<213> Artificial Sequence

<220>
<223> Reverse Primer Sequence For RhoA

<400> 14
tgtgctcatc attccgaaga 20

<210> 15
<211> 24
<212> DNA
<213> Artificial Sequence

<220>
<223> Forward Primer Sequence For Integrin betal

<400> 15
tgccaaatca tgtggagaat gtat 24

<210> 16
<211> 22
<212> DNA
<213> Artificial Sequence

<220>
<223> Reverse Primer Sequence For Integrin beta1

<400> 16

gtctgtggct cccctgatct ta 22

<210> 17
<211> 18
<212> DNA
<213> Artificial Sequence

<220>
<223> Forward Primer Sequence For Sox9

<400> 17
gctggcggat cagtaccc 18

<210> 18
<211> 18
<212> DNA
<213> Artificial Sequence

<220>
<223> Reverse Primer Sequence For Sox9

<400> 18
cgcggctggt acttgtaa 18

<210> 19
<211> 19
<212> DNA
<213> Artificial Sequence

<220>
<223> Forward Primer Sequence For COMP

<400> 19
ggcacattcc acgtgaaca 19

<210> 20
<211> 20
<212> DNA
<213> Artificial Sequence

<220>
<223> Reverse Primer Sequence For COMP

<400> 20
ggtttgcctg ccagtatgtc 20

## Description

### FIELD OF THE INVENTION

The invention refers to the field of tissue engineering, in particular to scaffolds, such as hydrogel scaffolds usable to replace tissues, such as an organ, bone or parts of it.

### BACKGROUND OF THE INVENTION

Tissue engineering techniques generally require the use of a temporary scaffold as a three-dimensional template for initial cell attachment and subsequent tissue formation. The ability of the scaffold to be metabolised by the body allows it to be gradually replaced by new cells to form functional tissue. As such, scaffold design is one of the most important aspects of tissue engineering.

A scaffold is expected to possess the following characteristics for use in tissue engineering: (i) a three-dimensional porous structure that allows cell/tissue growth and flow transport of nutrients and metabolic waste; (ii) biodegradable or bioresorbable with a controllable degradation and resorption rate to match cell/tissue growth *in vitro* and/or *in vivo*; (iii) conducive surface chemistry for cell attachment, proliferation, and differentiation; (iv) mechanical properties to match those of the tissues at the site of implantation; and (v) processability to form a variety of shapes and sizes for various applications.

Hydrogels have shown great promise as a scaffold for tissue engineering due to their tissue-like water content, good bio compatibility, and injectable accessibility for *in situ* grafting. Besides their use as scaffolds, hydrogels can additionally be used for cell delivery. Generally, cell conveyance with hydrogels consists of three steps: (i) suspending living cells in aqueous solution of hydrogel precursors, (ii) injecting the mixture to target sites and (iii) triggering the crosslinking (gelation) to form three-dimensional gel matrices *in situ.*

However, substantial challenges remain in the use of hydrogels as scaffold and cell delivery materials. For example, hydrogels have low cell affinity. Therefore, when they are used to encapsulate cells commonly used in regenerative medicine, such as fibroblasts, osteoblasts, endothelial, epithelial and smooth muscle cells, these anchorage dependent cells (ADC) do not spread out in the hydrogel framework but are constrained into a spheroidal shape, thereby leading to poor settlement and frequent occurrence of cell death. In addition, spatial confinement of cells within hydrogel bulk prevents cell migration and cell-cell interaction which are essential in mediating cell differentiation and tissue regeneration, as well as inhibiting cell aggregation which is particularly necessary for the reorganization of tissues such as cartilage and liver.

The patent US 2002/150604 Aldiscloses a scaffold for tissue engineering comprising bioabsorbable materials each material having a different rate of bioabsorption and a bioabsorbable binding gel which sets and binds the bioabsorbable materials in relation to each other and cells.

It is therefore an object of the present invention to provide compositions which can be used for the manufacture of scaffolds for tissue engineering which overcome at least some of the above disadvantages.

### SUMMARY OF THE INVENTION

In a first aspect, the invention provides a composition comprising a mixture of at least one degradable hydrogel and at least one kind of degradable and surface cross-linked particle as diclosed in claim 1.

In a second aspect, the invention provides a method of manufacturing a composition as disclosed in claim 9.

### BRIEF DESCRIPTION OF THE DRAWINGS

The invention will be better understood with reference to the detailed description when considered in conjunction with the non-limiting examples and the accompanying drawings, in which:

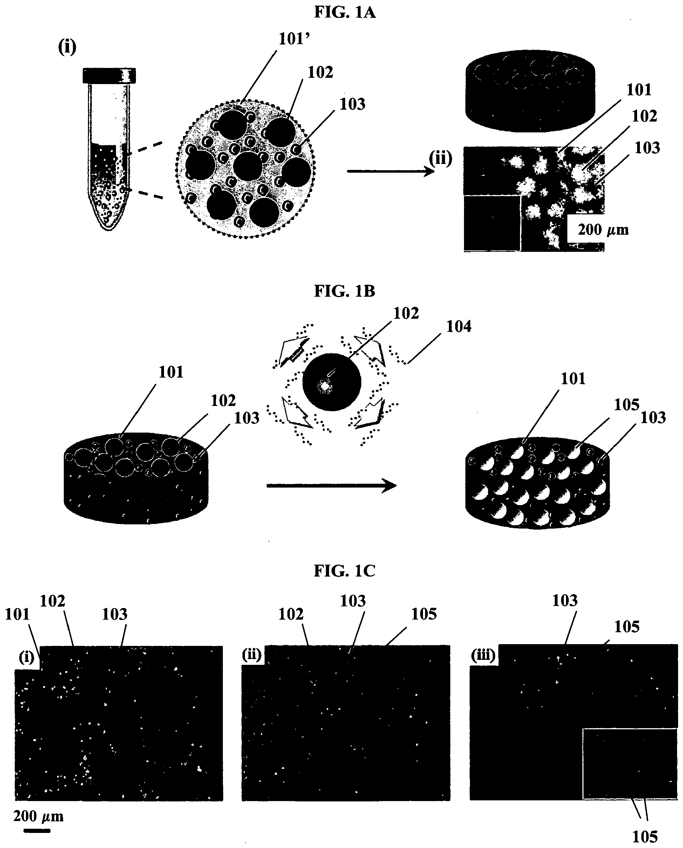

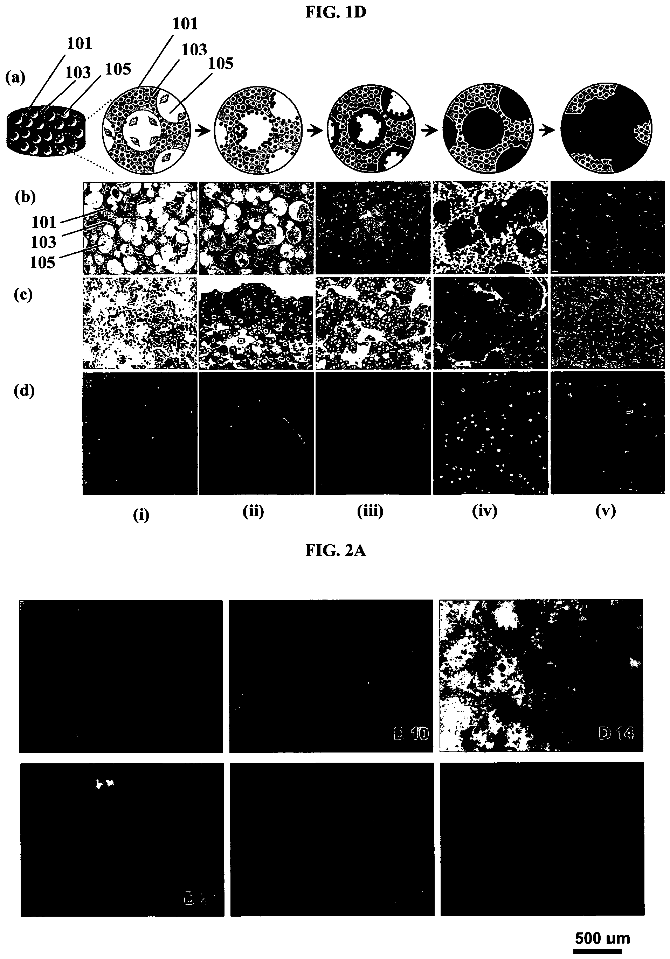

**Figure 1** is a schematic and histological illustration of a method to form a degradable hydrogel matrix comprising a cavity and a living cell. The inventors have put forth a model called the Phase Transfer Cell Culture (PTCC) model for the methodology according to the present invention.

**Figure 1A** shows (i) a schematic diagram of a composition comprising a degradable hydrogel precursor **101'**, a degradable and surface cross-linked particle **102**, and a living cell **103.** In this embodiment, living cells **103** and degradable and surface cross-linked particles **102** are suspended together in a degradable hydrogel precursor **101'** in an aqueous medium. Subsequently, the composition can be set or solidified to form a degradable hydrogel **101**, wherein the degradable hydrogel can encapsulate the degradable and surface cross-linked particles **102** and the living cells **103.** Also shown is (ii) an optical microscopy image, in which the degradable hydrogel **101**, degradable and surface cross-linked particle **102,** and living cell **103** can be seen.

**Figure 1B** is a schematic illustration of a process to form a cavity in a hydrogel matrix comprising a degradable hydrogel **101,** a degradable and surface cross-linked particle **102** and a living cell **103.** In this embodiment, a hydrogel matrix comprising degradable hydrogel **101,** degradable and surface cross-linked particles **102** and living cells **103** are subjected to certain conditions, which can cause the degradable and surface cross-linked particles **102** to degrade, forming degraded products **104.** These degraded products 104 of the degradable and surface cross-linked particles **102** can permeate through the hydrogel matrix, as represented by the outward pointing arrows. Consequently, cavities **105** can be formed in the area originally occupied by the degradable and surface cross-linked particles **102** in the hydrogel matrix.

**Figure 1C** shows optical microscopy images of a hydrogel matrix after an incubation time of (i) 30 mins, (ii) 2 hours and (iii) 2 days. In this specific embodiment, agarose was used as the degradable hydrogel **101,** gelatin microsphere (labeled with Rhodamine B, which gives off a red luminance) was used as the degradable and surface cross-linked particle **102,** and chondrocyte (stained with fluorescent calcein, which gives off a green luminance) was used as the living cell **103.** Gelatin microspheres were degraded using thermal degradation at 37 °C. As can be seen in (i), degradable and surface cross-linked particles **102** and living cells **103** are dispersed in the degradable hydrogel **101.** In (ii), it can be observed that some of the degradable and surface cross-linked particles **102** have degraded, leaving behind cavities **105.** As can be seen in (iii), most of the degradable and surface cross-linked particles **102** have degraded after 2 days of incubation, leaving behind cavities **105** in the area originally occupied by the degradable and surface cross-linked particles **102.** Also shown as an insert figure in (iii) is a scanning electron microscopy (SEM) image of the cavities **105** obtained.

**Figure 1D** is a schematic illustration and optical microscopy images of a process in which living cells **103** fill up cavities **105** within a degradable hydrogel matrix. Row (a) shows a schematic illustration of living cell movement as they are being incubated in the degradable hydrogel. The column panels from (i) to (v) show the various stages. In stage (i), living cells **103** are dispersed in the degradable hydrogel **101.** At this stage, the living cells **103** can proliferate in the hydrogel. In addition, the living cells **103** can migrate or move in the direction as shown by the arrows towards the cavities **105.** In stage (ii), the living cells **103** can sprout out of a hydrogel-cavity interface. In stage (iii), the living cells **103** can continue to grow and form neo-tissues, which can line the inner surface of the cavities **105.** As shown in stage (iv), the cavities **105** can eventually be filled up with neo-tissues formed by the living cells **103,** thereby forming islets, which can be spherical. In stage (v), the islets can fuse together, forming a network of interconnecting tissues, which can lead to formation of a scaffold for tissue engineering. The row panels from (b) to (d) show optical microscopy images of histological indication using (b) hematoxylin and eosin (H&E) stain in stage (i) to (iii) and (v), and Masson's Trichrome stain in stage (iv) which shows collagen production in blue (circled using dash line); (c) Safranin-O stain in stage (i) to (v) to show glycosaminoglycan (GAG) production in red (indicated using arrows), and (d) multiple fluorescence microscopy (MFM) using fluorescent DAPI (4',6-diamidino-2-phenylindole) stain to position the cell nuclei in purple, and green fluorescent calcein to stain the living cells in stage (i) and (iii), and immunohistochemical stain conjugating with green fluorescent chromophore to stage (ii), (iv) and (v) with type II collagen as the primary antibody. The staining results illustrated in Figure 1D show the high viability of the cells throughout the neo-tissue islet generation and the increasing total collage production from the cells in the microcavity cell (MCG) construct. With further cultivation, these scaffold-free islets act as neo-tissue cores and demonstrated strong tendency of expansion to fuse with each other and also with the isolated cells or colonies remaining in gel bulk so that a piece of maximally scaffold-free 3D macroscopic tissue was tangibly engineered. Driven by the outward mechano-orientation due to the biomechanical response between hydrogel scaffold and the cell colonies inside, cells proliferate fast, and then sprout into the formed cavities and outgrow, finally fuse to form a scaffold for tissue engineering.

**Figure 2A** shows optical microscopic images of chondrocytes cell culture in alginate hydrogel with cavities, which is formed using a method according to the present invention. The cells are incubated for a time period of 3, 10, 14, 20, 35 and 42 days (denoted by Dx, wherein x represents the number of days). The images show that the chondrocytes aggregated and formed cell colonies around the cavities, and finally the cavities were almost filled up by the increasing cell clusters.

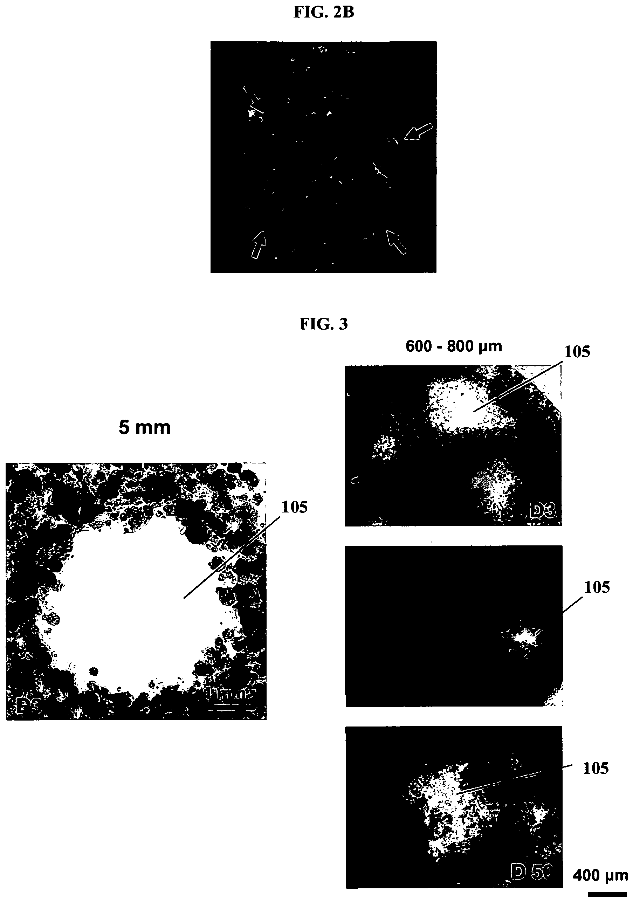

**Figure 2B** shows an optical microscopic image of chondrocytes cells which was cultured for 28 days in poly(ethylene glycol) hydrogel-diacrylate (PEGDA) hydrogel with cavities formed using a method according to the present invention. The image shows that a cell colony was formed which filled the cavity (boundary of cell colony is defined by arrows).

**Figure 3** shows chondrocytes in agarose with cavity sizes ranging from 400 μm to 5 mm. The chondrocytes were incubated for 3, 28 and 50 days (denoted by Dx, wherein x represents the number of days). The results show that cell colony formation in the cavities occurred.

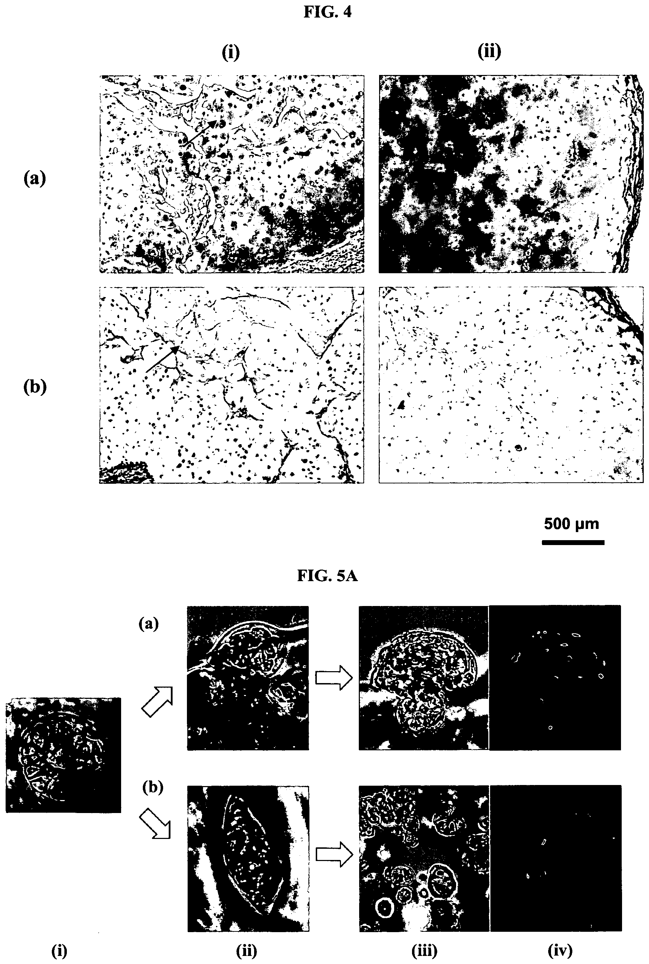

**Figure 4** are optical microscopy images of hydrogel implanted subcutaneously in nude mice for 1 week. Column (i) shows a hydrogel matrix formed according to a method of the present invention, using alginate as the degradable hydrogel, gelatin microspheres as the degradable and surface cross-linked particle and chondrocytes as the living cell. Column (ii) shows the control, using a conventional hydrogel matrix formed using alginate as the hydrogel and chondrocytes as the living cell. The samples in row (a) are stained with hematoxylin and eosin (H&E), and the samples in row (b) are stained with Masson's Trichrome. In row (a) the arrow indicates cell colonies and in row (b) the arrow indicates an area of densely collagen secretion. The images show that the cavities in the hydrogel matrix according to the present invention were filled up with extracellular matrix comprising collagen (arrow in row (b)). As shown by Masson staining, the collagen density in the hydrogel matrix according to the present invention was much higher than that in the control.

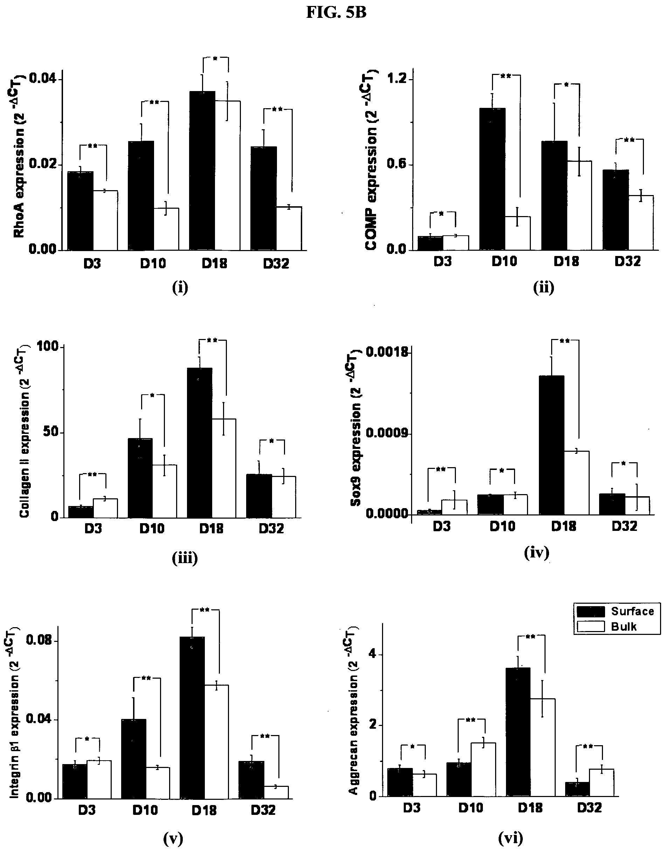

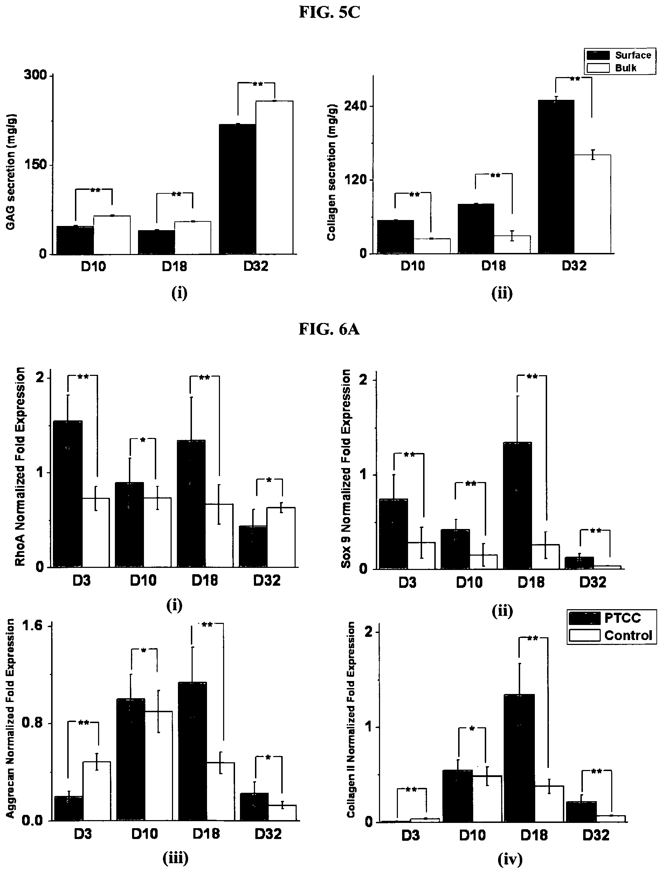

**Figure 5** are optical microscopy images and graphs comparing cell growth behavior in a hydrogel-air interface, and within the bulk of a hydrogel. Agarose was used as the hydrogel and chondrocytes as the living cell in both cases. This experiment was carried out to demonstrate tendency of living cells to migrate to the hydrogel-air interface, whereby the living cells can proliferate and grow at a faster rate compared to living cells located in the bulk of a hydrogel.

**Figure 5A** shows microscopy images of cell interaction in plain agarose hydrogel in hydrogel-air interface (shown in row (a)), and within the bulk of a hydrogel (shown in row (b)). The column panels from (i) to (ii) show continuous stages of cell growth using an incubation time of 3 and 12 days respectively. The living cell used was chondrocyte. In the first two columns (i) and (ii), optical microscopy was used to image cell behavior. In the last two columns (iii) and (iv), optical and fluorescent modes provided by laser confocal microscope (LCM) were used. Immunohistochemical stain of neural cell adhesion molecules (NCAM) conjugated with fluorescein isothiocyanate (FITC) was carried out for (iii) and (iv). As shown in (i), living cells can form spherical colonies in a hydrogel. In column (ii), the living cells located near the hydrogel-air interface can sprout out from the hydrogel and grow at the hydrogel-air interface as shown in (a), whereas in (b), the cells show a constraint morphology as they are surrounded by hydrogel, and can only grow in the regions in which pressure exerted by the growing cells is greater than pressure exerted by the hydrogel gel matrix on the cells. As can be seen from the magnified images in (iii) and (iv), the living cells located near the hydrogel-air interface proliferate at a greater rate than that of living cells located within the bulk of the hydrogel. The shrinking of the cell colony of row (b) is caused by the restriction of the hydrogel bulk.

**Figure 5B** are graphs showing analysis of the expression of relevant cartilaginous markers of (i) RhoA, (ii) COMP, (iii) Collagen Type II, (iv) Sox 9, (v) Integrin $\beta$1 and (vi) Aggrecan at transcriptional level from porcine chondrocytes in hydrogel-surface (grey) and hydrogel-bulk (white) by quantitative real-time polymerase chain reaction (RT-PCR) (n = 3) after 3, 10, 18 and 32 days (denoted by Dx, wherein x represent number of days). The values represent the levels of expression relative to that of the internal control gene (GAPDH) and are expressed as means $\pm$ standard deviations.

**Figure 5C** are graphs showing secretion of extracellular matrix (ECM) in gel-surface (grey) and gel-bulk (white) in plain agarose gel: secretion of (i) glycosaminoglycan (GAG) and (ii) total collagen as a function of culture time at 10, 18 and 32 days (denoted by Dx, wherein x represent number of days). The values have been normalized by the dry weight of the separated parts, and represent the means $\pm$ standard deviations. (The notation * indicates that differences between different constructs at the same culture time are not significant ($p > 0.05$), and the notation ** indicates that differences between different constructs at the same culture time are significant ($p < 0.05$).)

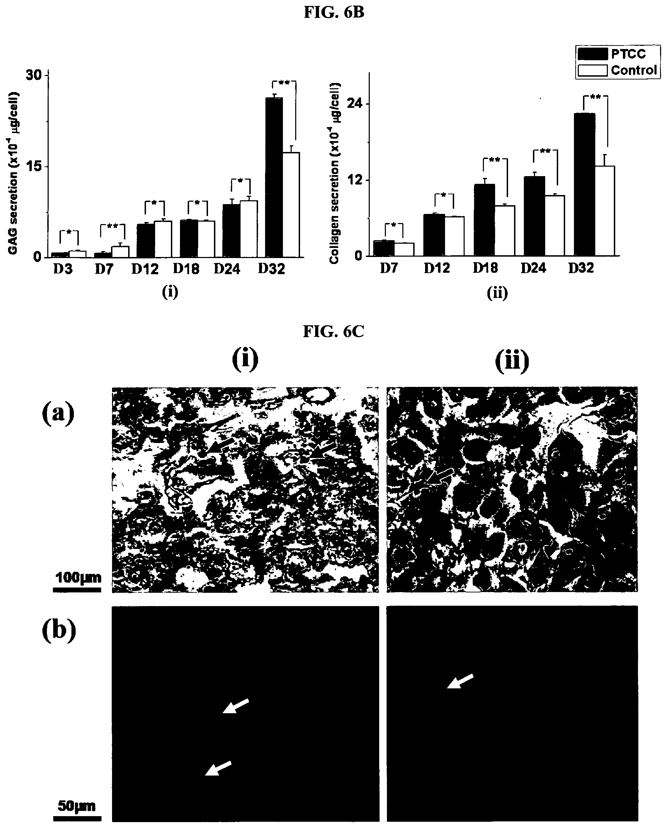

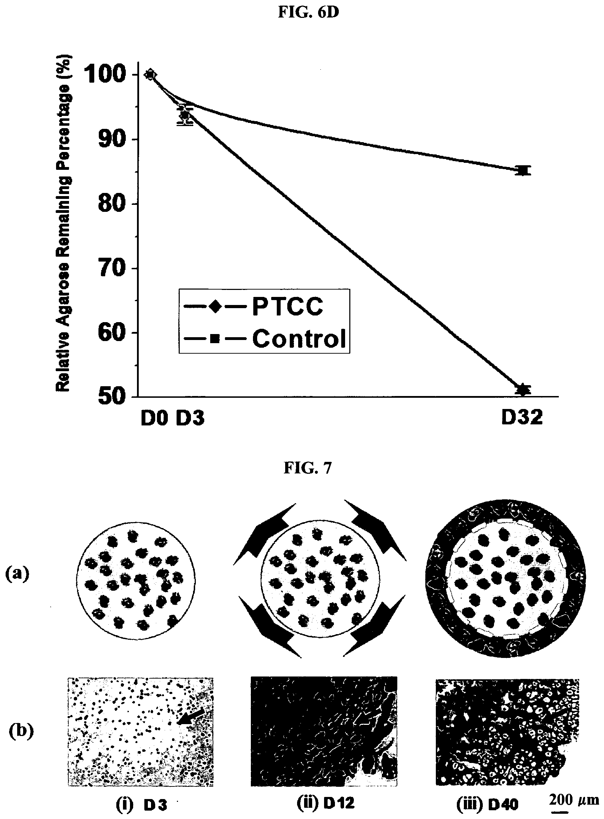

**Figure 6** are graphs and optical microscopy images comparing chondrocytes cell culture in agarose hydrogel with cavities, which is formed using a method according to the present invention, and control samples of chondrocytes cell culture in conventional agarose hydrogel.

**Figure 6A** are graphs showing analysis of the expression of relevant cartilaginous markers (i) RhoA, (ii) Sox 9, (iii) Aggrecan and (iv) Collagen Type II at transcriptional level from samples of porcine chondrocytes in agarose hydrogel with cavities formed using a method according to the present invention (denoted as "PTCC", shown in grey), and control samples of chondrocytes in conventional agarose hydrogel (denoted as "Control", shown in white) by quantitative real-time polymerase chain reaction (n = 3) after 3, 10, 18 and 32 days (denoted by Dx, wherein x represents number of days). The values represent the levels of expression relative to that of two internal control genes (HPRT and RPL4). The values represent the means $\pm$ standard deviations.

**Figure 6B** are graphs investigating secretion of extracellular matrix (ECM) (i) glycosaminoglycan (GAG) and (ii) total collagen in samples of porcine chondrocytes in agarose hydrogel with cavities formed using a method according to the present invention (denoted as "PTCC", shown in grey) and control samples of chondrocytes in conventional agarose hydrogel (denoted as "Control", shown in white) as a function of culture time at 3, 7, 12, 18, 24 and 32 days (denoted by Dx, wherein x represent number of days).

**Figure 6C** are optical microscopy images showing histological evaluation of (i) samples of porcine chondrocytes in agarose hydrogel with cavities formed using a method according to the present invention cultured *in vivo* for 40 days, and (ii) control samples of chondrocytes in conventional agarose hydrogel. In row (a), Safranin-O stain is applied to reveal positive glycosaminoglycan (GAG) production in red (indicated using arrows). In row (b), immunohistochemical stain conjugating with green fluorescent chromophore is applied to image the samples with primary antibody of type II collagen (indicated using arrows).

**Figure 6D** is a graph showing determination of agarose content inside the cell-laden constructs along with the passage of *in vitro* culture time. The kinetics of agarose weight change is quantified with relative agarose remaining percentage till 32 days (denoted by Dx, wherein x represent number of days). The curves are marked as cyan and magenta colour respectively for phase transfer cell culture and control samples. (The notation * indicates that differences between different constructs at the same culture time are not significant ($p > 0.05$) and the notation ** indicates that differences between different constructs at the same culture time are significant ($p < 0.05$).)

**Figure 7** is a schematic (shown in row (a)) and optical microscopy images (shown in row (b)) of Edge Flourish (EF) model in plain agarose hydrogel. In histological indication, positive glycosaminoglycan (GAG) production is demonstrated with red Safranin-O stain. This EF model is put forth by the inventors of the present application to explain and simulate the tendency of living cells to migrate to the hydrogel-air interface, whereby the living cells can proliferate and grow at a faster rate compared to living cells located in the bulk of a hydrogel. This phenomenon is also demonstrated in **Figure 5.** In row (a), cells are encapsulated within a hydrogel as shown in (i). The inventors have observed that there is a tendency for cells to migrate to the external surface of a hydrogel, as shown by the outward pointing arrows in (ii). This can lead to neo-tissue formation around the hydrogel, as shown in (iii). In row (b), the corresponding image shows cell seeding on Day 3 in (i), followed by cells outgrowth on Day 12, and neo-tissue formation around the gel on Day 40 (denoted by Dx, wherein x represent number of days).

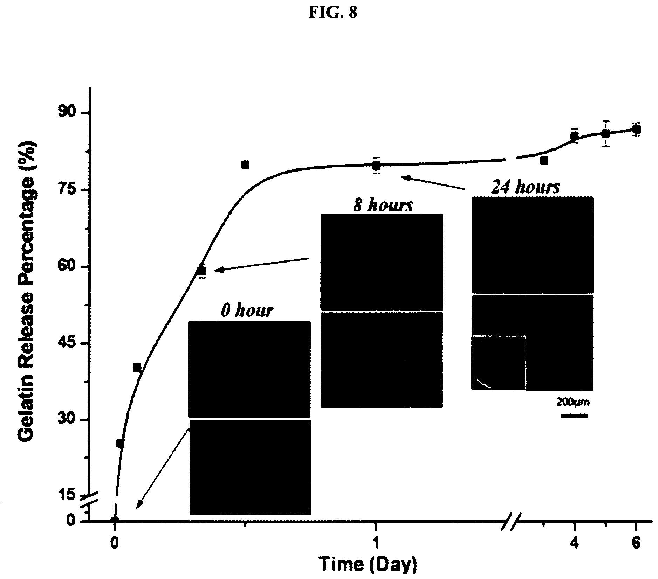

**Figure 8** shows kinetics of cavity creation quantified with gelatin release percentage over a time period of 6 days. The selected time points of detection are respectively 0, 8, and 24 hours and about 80% gelatin has been released within 24 hours. The graph shows that about 75% of the gelatin is degraded in 1 day, and about 85% of the gelatin is degraded after 6 days. Within the graph are inserts of optical microscopic images at time of 0, 8 and 24 hours respectively. The images show the disappearance of red luminance of gelatin spheres (pre-labeled with Rhodamine B fluorescent dye) over time.

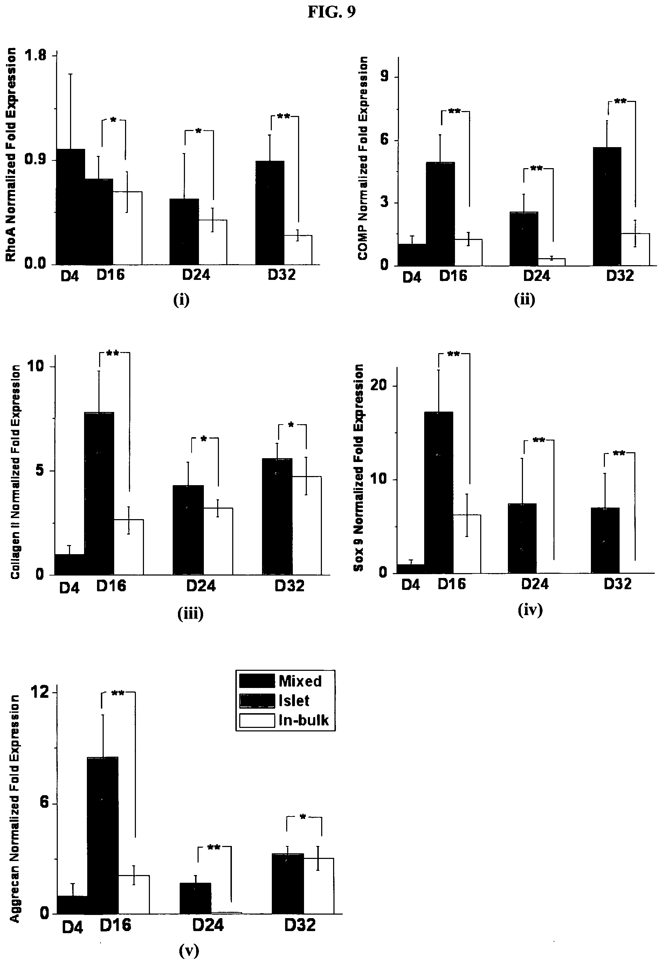

**Figure 9** are graphs showing analysis of the expression of relevant cartilaginous markers (i) RhoA, (ii) COMP, (iii) Collagen Type II, (iv) Sox 9 and (v) Aggrecan at transcriptional level from porcine chondrocytes in alginate hydrogel with cavities formed using a method according to the present invention. Aggregate cell islets (grey), individual cells in bulk (white) are distinguished by quantitative real-time polymerase chain reaction ($n = 3$) after 4 (initial stage), 16, 24 and 32 days respectively days (denoted by Dx, wherein x represent number of days). The mixed cells at initial stage (4 days) are indicated in black. The values represent the levels of expression relative to that of the internal control gene TBP1. The values represent the means $\pm$ standard deviations. (The notation * indicates differences between different constructs at the same culture time are not significant ($p > 0.05$) and the notation ** indicates differences between different constructs at the same culture time are significant ($p < 0.05$).)

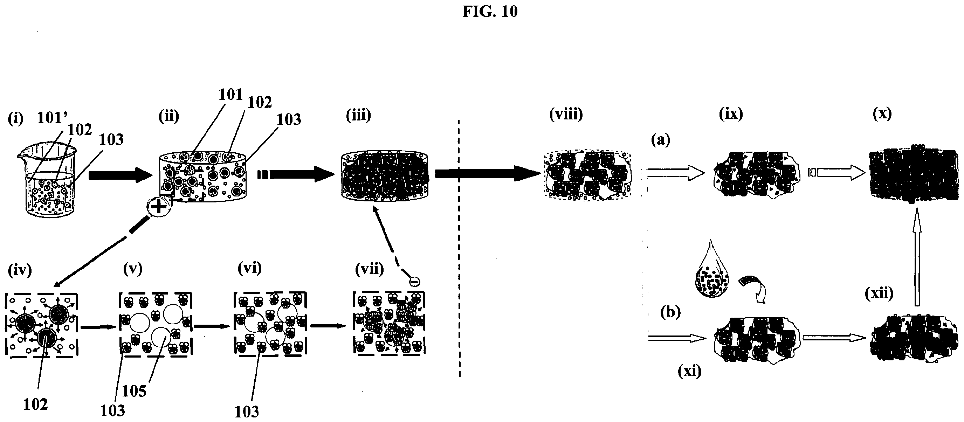

**Figure 10** is a schematic illustration of scaffold for tissue engineering formation. Stage (i) to (vii) have previously been explained in **Figure 1,** i.e. in stage (i), a composition comprising a degradable hydrogel precursor **101’,** a degradable and surface cross-linked particle **102,** and a living cell **103** is provided. In this embodiment, living cells **103** and degradable and surface cross-linked particles **102** are suspended together in a degradable hydrogel precursor **101’** in an aqueous medium. Subsequently, the composition can be set or solidified to form a degradable hydrogel **101,** wherein the degradable hydrogel can encapsulate the degradable and surface cross-linked particles **102** and the living cells **103,** as shown in stage (ii). The hydrogel matrix comprising degradable hydrogel **101,** degradable and surface cross-linked particles **102** and living cells **103** are subjected to certain conditions, which can cause the degradable and surface cross-linked particles **102** to degrade, forming degraded products which can permeate through the hydrogel matrix, as represented by the outward pointing arrows in stage (iv). Consequently, cavities **105** can be formed in the area originally occupied by the degradable and surface cross-linked particles **102** in the hydrogel matrix as shown in stage (v). In stage (vi), living cells **103** dispersed in the degradable hyrogel **101** can proliferate in the hydrogel. In addition, the living cells **103** can migrate or move towards the cavities. In stage (vii), the living cells **103** can continue to grow and form neo-tissues, which can eventually fill up the cavities thereby forming islets, which can be spherical. In stage (iii), the islets can fuse together, forming a network of interconnecting tissues. In stage (viii), the hydrogel is degraded from the hydrogel matrix comprising the generated fused tissues from stage (iii). Subsequently, a cartilage-like tissue can be formed from either of two pathways, i.e. (a) by culturing without or (b) with adding cells exogenously.

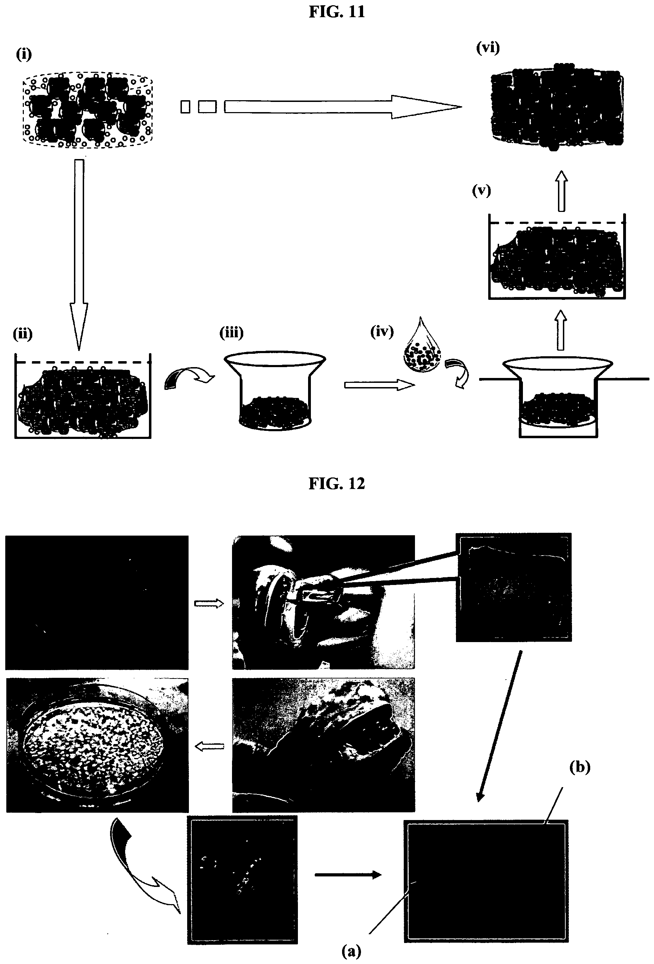

**Figure 11** is a schematic illustration of a method to form a scaffold for tissue engineering by adding extra cells. As shown in the figure, the tissue is cultured after gel detachment in plate for 7 days to enhance stability of the tissue as shown in (ii). Subsequently, the cultured tissue is transferred to an insert, followed by centrifuge to remove surrounding medium as shown in (iii). Cells are then added into the tissue, followed by culturing it in the insert system for 7 days as shown in (iv), and then transferring the tissue to a normal culture plate for culturing until cartilage-like tissue are obtained as shown in (v) and (vi).

**Figure 12** is a photograph illustration of the formation of scaffold for tissue engineering, as well as comparison of a scaffold for tissue engineering according to an embodiment of the present invention (denoted by (b)), and a native cartilage chip (denoted by (a)), i.e. part of a natural cartilage removed from a bone used in the experiment. The results show that the scaffold obtained from an embodiment of the present invention closely resembles the native cartilage chip obtained from the bone.

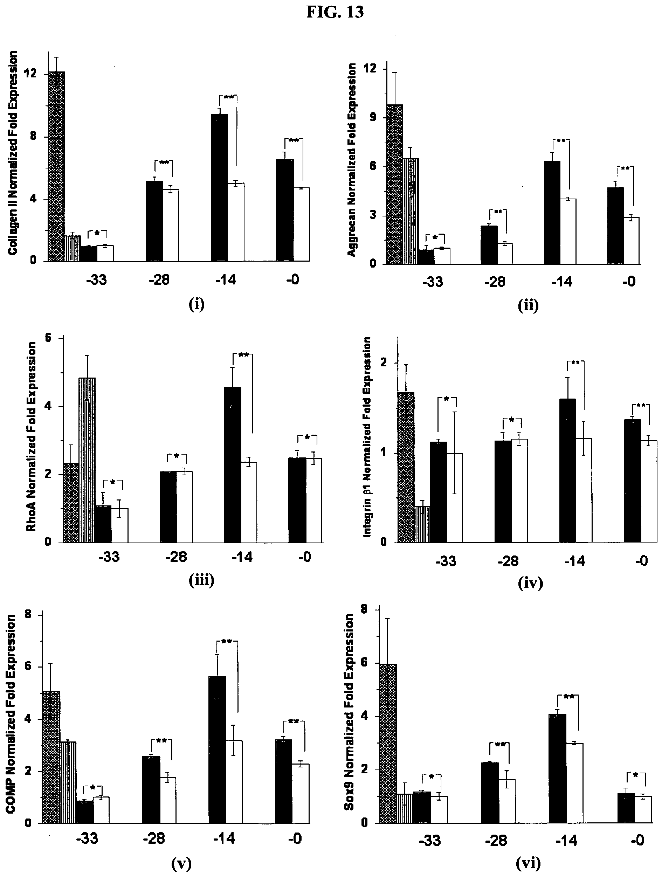

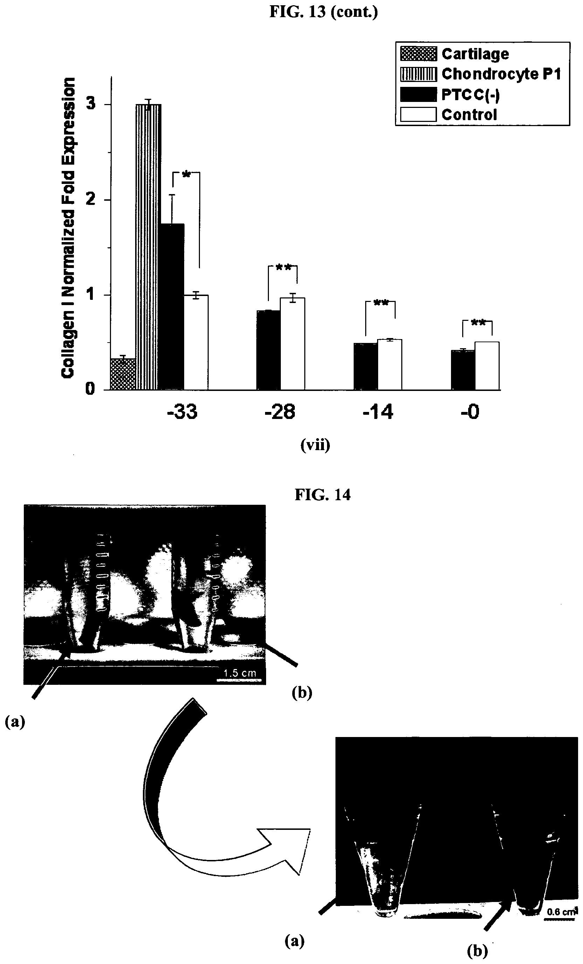

**Figure 13** are graphs showing an analysis of the expression of relevant cartilaginous markers (i) Collagen Type II, (ii) Aggrecan, (iii) RhoA, (iv) Integrin $\beta$1, (v) COMP, (vi) Sox 9, and (vii) non-cartilaginous marker (Collagen Type I) at transcriptional level using samples from porcine chondrocytes in alginate hydrogel with cavities formed using a method according to the present invention (denoted as "PTCC") and control samples of chondrocytes in conventional alginate hydrogel (denoted as "Control"), as well as both native Cartilage (denoted as "Cartilage") and Chondrocyte Passage 1 (denoted as "Chondrocyte P1") samples by quantitative real-time PCR (n = 3) after 2, 7, 21 and 35 days. The x-axis shows the culture time in days. The values represent the levels of expression relative to that of the internal control gene (TBP1) and are expressed as means $\pm$ standard deviations. PTCC samples showed better performance than Control. (The notation * indicates differences between different constructs at the same culture time are not significant ($p > 0.05$) and the notation ** indicates differences between different constructs at the same culture time are significant ($p < 0.05$).)

**Figure 14** shows photographs of results of (a) PTCC sample scaffold for tissue engineering according to an embodiment of the present invention and (b) control sample of chondrocytes in conventional alginate hydrogel after the detachment of alginate hydrogels (indicated by arrows). As shown, after alginate was detached, scaffold sample according to an embodiment of the present invention could still hold its whole shape as an integral construct.

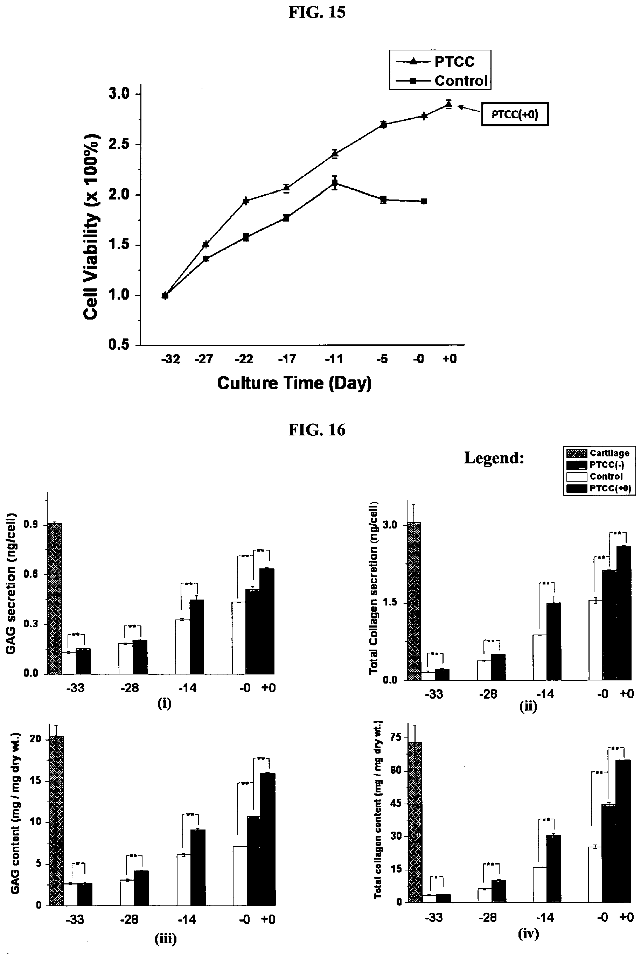

**Figure 15** is a graph showing cell proliferation in (a) scaffold for tissue engineering according to an embodiment of the present invention (denoted as "PTCC") and (b) control sample of chondrocytes in conventional alginate hydrogel (denoted as "Control") examined with WST-1 colorimetric assay. As shown, cell viability in the PTCC samples (about 280%) was significant better by about 1.6 times than that in control samples (about 180%).

**Figure 16** are graphs showing investigation of the secretion of extracellular matrix (ECM) of glycosaminoglycan (GAG) and total collagen in samples from porcine chondrocytes in alginate hydrogel with cavities formed using a method according to the present invention (denoted as "PTCC") and control samples of chondrocytes in conventional alginate hydrogel (denoted as "Control") as a function of culture time at 2, 7, 21, and 35 days. (i) and (iii) shows graphs of GAG expressed in terms of GAG secretion (ng/cell) and GAG content (mg/mg dry weight) respectively. (ii) and (iv) shows graphs of total collagen expressed in terms of total collagen secretion (ng/cell) and total collagen content (mg/mg dry weight) respectively. Moreover, the ECM secretion in PTCC samples which underwent gel detachment was also examined. As shown, regarding the ECM secretion amount either in GAG or total collagen, PTCC samples performed higher ECM secretion than control over time. (The notation * indicates differences between different constructs at the same culture time are not significant ($p > 0.05$) and the notation ** indicates differences between different constructs at the same culture time are significant ($p < 0.05$).)

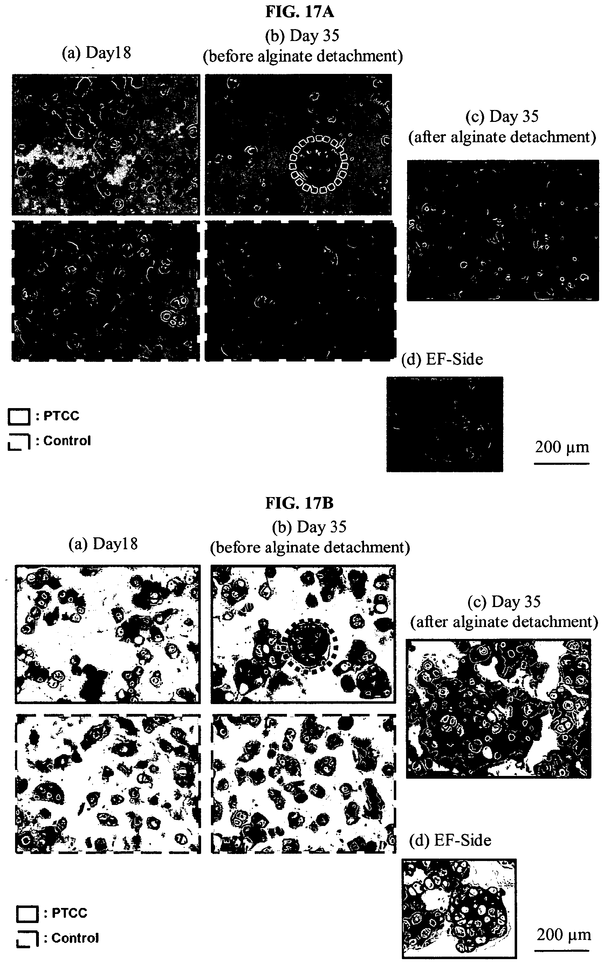

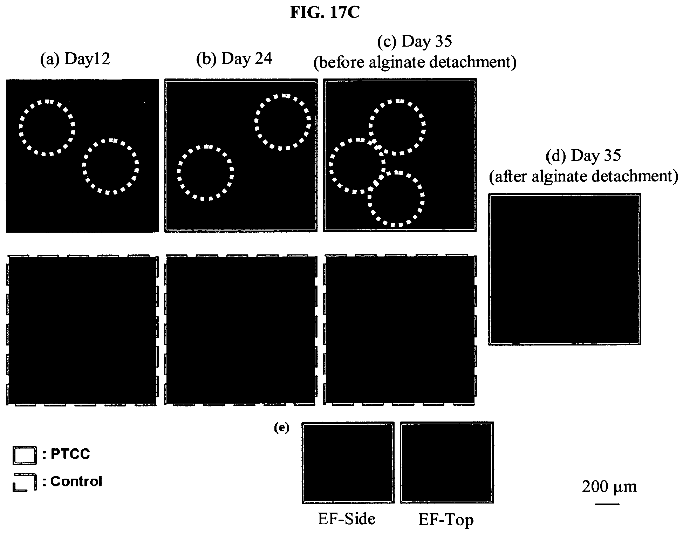

**Figure 17** shows optical microscope images of histological evaluation of samples from porcine chondrocytes in alginate hydrogel with cavities formed using a method according to the present invention (denoted as "PTCC") cultured for 35 days in contrast with control samples of chondrocytes in conventional alginate hydrogel (denoted as "Control"). EF-side refers to the edge flourish side which is from the edge of the hydrogel and not from the hydrogel cavity.

**Figure 17A** shows application of hematoxylin and eosin (H&E) stain to survey the histological evolution.

**Figure 17B** shows application of Safranin-O stain to reveal positive glycosaminoglycan (GAG) production in red.

**Figure 17C** shows Live/Dead staining (mainly stained by green fluorescent calcein) was applied to test chondrocyte viability in the hydrogel samples. As shown by the areas highlighted with dashed circles in the images, the process of cell sprouting with subsequent cell colony filling-up and tissue fusing was reproduced in PTCC samples, in contrast to the results of the Control samples, in which such cell colonies and tissue fusing were not observed.

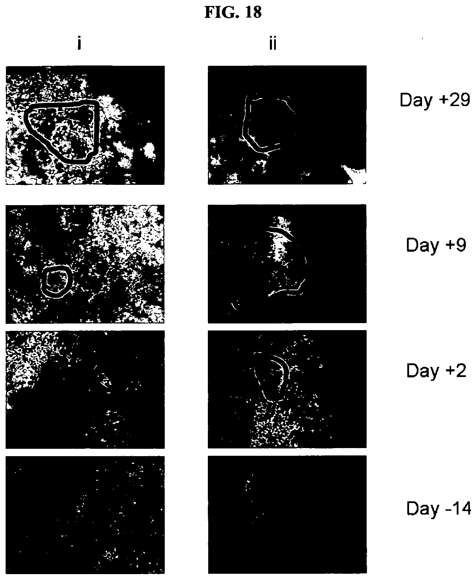

**Figure 18** shows optical microscope images of PTCC tissue development, which underwent the detachment of alginate hydrogels and subsequently cultured either (i) with or (ii) without extra addition of additional cells. As shown, more and more cell colonies appeared and fused together within the PTCC tissues (a few such areas are exemplarily highlighted with a circle) as a function of culture time, both in the above two kinds of tissues.

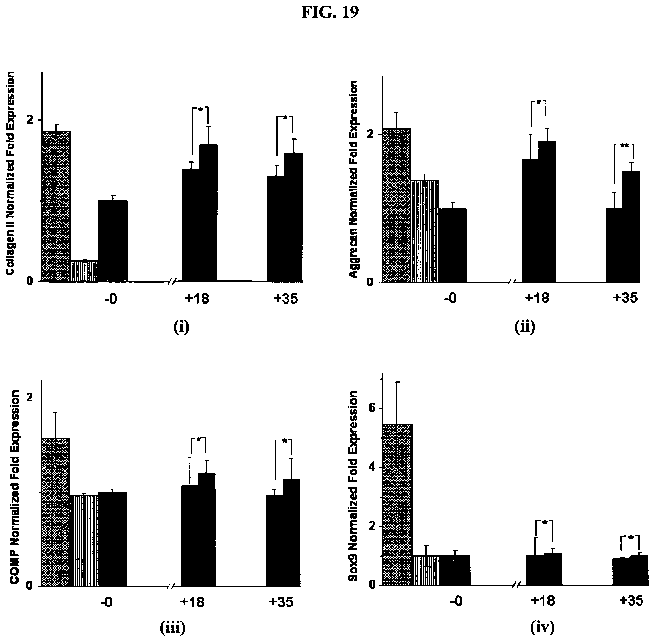

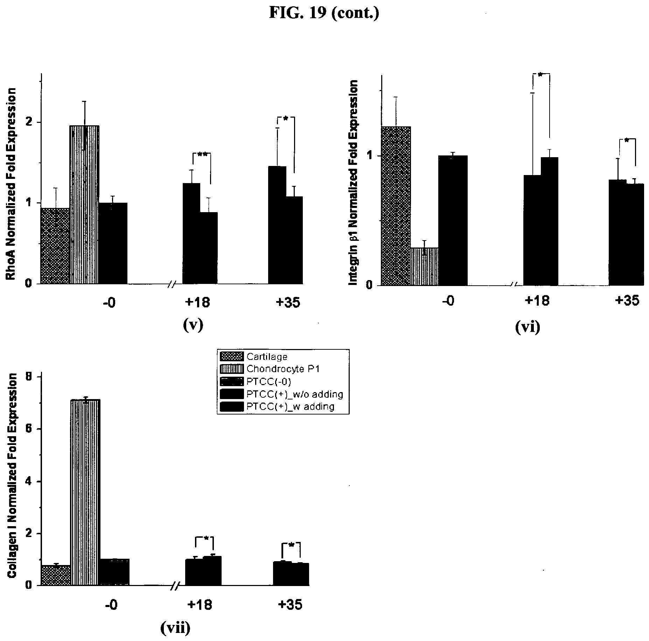

**Figure 19** are graphs showing analysis of the expression of relevant cartilaginous markers (i) Collagen Type II, (ii) Aggrecan, (iii) COMP, (iv) Sox 9, (v) RhoA, (vi) Integrin ($\beta$1) and (vii) non-cartilaginous marker (Collagen Type I) at transcriptional level from porcine chondrocytes that were encapsulated in scaffold according to an embodiment of the present invention with (denoted by "PTCC_w adding") or without (denoted by "PTCC_w/o adding") adding cells, as well as native cartilage (denoted by "Cartilage") and Chondrocyte Passage 1 (denoted by "Chondrocyte P1") samples by quantitative real-time PCR (n = 3) after 2, 7, 21 and 35 days (PTCC(-0) samples point to the samples cultured for 35 days before gel-detachment). The values represent the levels of expression relative to that of the internal control gene (TBP1) and are expressed as means $\pm$ standard deviations. As shown, the living scaffolds performed stable gene expression even after gel detachment. (The notation * indicates differences between different constructs at the same culture time are not significant ($p > 0.05$) and the notation ** indicates differences between different constructs at the same culture time are significant ($p < 0.05$).)

## DETAILED DESCRIPTION OF THE PRESENT INVENTION

The invention is based on the finding that cavities can be formed in a hydrogel matrix using degradable and surface cross-linked particles that degrade at a faster rate compared to the hydrogel matrix at a given condition. Therefore, by subjecting the hydrogel matrix comprising the degradable and surface cross-linked particles to conditions at which the particles preferentially degrade compared to the hydrogel matrix, cavities can be formed in the hydrogel matrix. The composition comprising the hydrogel and degradable and surface cross-linked particles can further comprise one or more species of living cells. The cells can be added together with the degradable and surface cross-linked particles to the hydrogel solution. The living cells which are present in the hydrogel matrix of the present invention can proliferate at a faster rate compared to a hydrogel matrix which has no cavities, and can continue to grow within the cavities formed by degradation of the degradable and surface cross-linked particle thereby completely filling the cavities. The hydrogel matrix comprising the living cells can be removed after the cells have filled the cavities, thereby forming a scaffold for tissue engineering which is made from the living cells. Additional living cells may be added to the scaffold and incubated such that a tissue or organ can be formed using the scaffold.

In a first aspect, the invention provides a composition comprising one or more species of living cells, and a mixture of at; least one degradable hydrogel and at least one kind of degradable and surface cross-linked particle, wherein the at least one kind of degradable and surface cross-linked particle comprises a material which degrades faster than the at least one degradable hydrogel, wherein the at least one kind of degradable and surface cross-linked particle has a surface comprising additional reactive groups to bind the one or more species of living cells to its surface, and wherein the one or more species of living cells bound to the surface of the at least one kind of degradable and surface cross-linked particle are comprised in the at least one degradable hydrogel, and wherein the at least one degradable hydrogel of the mixture is formable to a hydrogel matrix.

As used in this application, the term "hydrogel" refers to a broad class of polymeric materials, that can be natural or synthetic, which have an affinity for an aqueous medium, and can absorb large amounts of the aqueous medium, but which do not normally dissolve in the aqueous medium. The term "aqueous medium" as used herein refers to water or a solution based primarily on water such as phosphate buffered saline (PBS), or water containing a salt dissolved therein. The term aqueous medium can also refer to a cell culture medium. The term "cell culture medium" refers to any liquid medium which enables cells proliferation. Growth media are known in the art and can be selected depending of the type of cell to be grown. For example, a growth medium for use in growing mammalian cells is Dulbecco's Modified Eagle Medium (DMEM) which can be supplemented with heat inactivated fetal bovine serum.

A hydrogel is characterized by a high permeability for exchange of nutrients necessary for cell proliferation. The physical properties of hydro gels are similar to native tissue and hydrogels can be used to encapsulate cells in the hydrogel matrix formed upon gelation. In one embodiment, an injectable hydrogel is used which can be injected. Also, in one embodiment the mixture comprised of at least one degradable hydrogel and at least one kind of degradable and surface cross-linked particle or the mixture of at least one degradable hydrogel, at least one kind of degradable and surface cross-linked particle and at least one type of cell species is injectable.

Generally, a hydrogel can be formed by using at least one or one or more types of hydrogel precursor, and setting or solidifying the one or more types of hydrogel precursor in an aqueous solution to form a three-dimensional network, wherein formation of the three-dimensional network can cause the one or more types of hydrogel precursor to gel. The term "hydrogel precursor" refers to any chemical compound that can be used to make a hydrogel. Examples of hydrogel precursors include, but not limited to, a natural polymer, a hydrophilic monomer, a hydrophilic polymer, a hydrophilic copolymer formed from a monomer and a polymer. Polymer or scaffold materials which are not hydrogels are not used in the composition of the present invention.

In some embodiments, the hydrogel precursor can be a natural polymer. The natural polymer can form a three-dimensional network in an aqueous medium to form a hydrogel. A "natural polymer" refers a polymeric material that can be found in nature. Examples of a natural polymer include, but are not limited to, polysaccharide, glycosaminoglycan, protein, peptide and polypeptide. Polysaccharides are carbohydrates which can be hydrolyzed to two or more monosaccharide molecules. They can contain a backbone of repeating carbohydrate i.e. sugar unit. Examples of polysaccharides include, but are not limited to, alginate, agarose, chitosan, dextran, starch, and gellan gum. Glycosaminoglycans are polysaccharides containing amino sugars as a component. Examples of glycosaminoglycans include, but are not limited to, hyaluronic acid, chondroitin sulfate, dermatin sulfate, keratin sulfate, dextran sulfate, heparin sulfate, heparin, glucuronic acid, iduronic acid, galactose, galactosamine, and glucosamine. As used herein, the terms protein, peptide and polypeptide are used interchangeably. Therefore, for purposes of this application, a protein refers to proteins, protein fragments, peptides and polypeptides. Proteins belong to the broad class of polypeptides, and refer to organic compounds molecules containing more than one amino acid (which include native and non-native amino acid monomers). Proteins have diverse biological functions and can be classified into five major categories, i.e. structural proteins such as collagen, catalytic proteins such as enzymes, transport proteins such as hemoglobin, regulatory proteins such as hormones, and protective proteins such as antibodies and thrombin. Other examples of proteins include, but are not limited to, fibronectin, gelatin, fibrin, pectins, albumin, ovalbumin, and polyamino acids.

In some embodiments, the hydrogel precursor can be a hydrophilic monomer. As used herein, a hydrophilic monomer refers to any monomer which, when polymerized, yields a hydrophilic polymer capable of forming a hydrogel when contacted with an aqueous medium such as water. In some embodiments, a hydrophilic monomer can contain a functional group in the polymer backbone or as lateral chains. The term "functional group" as used herein refers to a chemical moiety which exhibits bond formation capability. Examples of functional group include, but are not limited to, hydroxyl (-OH), carboxyl (-COOH), amide (-CONH-), thiol (-SH), or sulfonic (-$SO_3H$) groups. Examples of hydrophilic monomers include, but are not limited to, hydroxyl-containing monomers such as 2-hydroxyethyl methacrylate, 2-hydroxyethyl acrylate, 2-hydroxyethyl methacrylamide, 2-hydroxyethyl acrylamide, N-2-hydroxyethyl vinyl carbamate, 2-hydroxyethyl vinyl carbonate, 2-hydroxypropyl methacrylate, hydroxyhexyl methacrylate and hydroxyoctyl methacrylate; carboxyl-containing monomers such as acrylic acid, methacrylic acid, itaconic acid, fumaric acid, crotonic acid, maleic acid and salts thereof, esters containing free carboxyl groups of unsaturated polycarboxylic acids, such as monomethyl maleate ester, monoethyl maleate ester, monomethyl fumarate ester, monoethyl fumarate ester and salts thereof; amide-containing monomers such as (meth)acrylamide, crotonic amide, cinnamic amide, maleic diamide and fumaric diamide; thiol-containing monomers such as methanethiole, ethanethiol, 1-propanethiol, butanethiol, tert-butyl mercaptan, and pentanethiols; sulfonic acid-containing monomers such as p-styrenesulfonic acid, vinylsulfonic acid, p-a-methylstyrenesulfonic acid, isoprene sulfonide and salts thereof.

In some embodiments, a hydrogel precursor can be a hydrophilic polymer. The hydrophilic polymer can be a polymer that is made up of any one of the above mentioned hydrophilic monomer, and which can be formed from any reaction such as, but not limited to, free radical polymerization, condensation polymerization, anionic or cationic polymerization, or step growth polymerization. For example, a hydrophilic monomer such as ethylene glycol can undergo anionic or cationic polymerization, depending on the type of catalyst used, to form poly(ethylene glycol) which is a hydrophilic polymer. The hydrophilic polymer can also be obtained by chemical modification of an existing polymer. For example, a functional group can be added or altered on polymeric chains such that the resultant polymer is made hydrophilic.

In some embodiments, a hydrogel precursor can be a hydrophilic copolymer. The hydrophilic copolymer can be formed from a hydrophilic polymer and a monomer which can be hydrophilic, hydrophobic or amphiphilic. In some embodiments, a hydrophilic copolymer can be formed from a hydrophilic monomer and a polymer which can be hydrophilic, hydrophobic or amphiphilic. For example, a hydrophobic monomer can react with a functional group present on a hydrophilic polymer to form a hydrophilic copolymer.

The one of more type of hydrogel precursors can set or solidify in an aqueous medium to form a three-dimensional network, wherein formation of the three-dimensional network can cause the one or more types of hydrogel precursor to gel. For example, a hydrogel can be formed by physical bonding such as self assembly, or chemical bonding such as cross-linking, of one or more types of hydrogel precursors in an aqueous medium.

In some embodiments, a hydrogel can be formed by self-assembly of one or more types of hydrogel precursors in an aqueous medium. The term "self-assembly" refers to a process of spontaneous organization of components of a higher order structure by reliance on the attraction of the components for each other, and without chemical bond formation between the components. For example, polymer chains can interact with each other via any one of hydrophobic forces, hydrogen bonding, Van der Waals interaction, electrostatic forces, or polymer chain entanglement, induced on the polymer chains, such that the polymer chains can aggregate or coagulate in an aqueous medium, which can form a three-dimensional network, thereby entrapping molecules of water to form a hydrogel.

In some embodiments, a hydrogel can be formed by chemical bonding between one or more types of hydrogel precursors in an aqueous medium. For example, when the hydrogel precursor is a hydrophilic polymer, the polymeric chains can be cross-linked using a suitable cross-linking agent to form a three-dimensional network, which can entrap water molecules to form a hydrogel. The term "cross-link" as used herein refers to an interconnection between polymer chains via chemical bonding, such as, but not limited to, covalent bonding, ionic bonding, or affinity interactions (e.g. ligand/receptor interactions, antibody/antigen interactions, etc.). The chemical cross-linking can be carried out by reactions, such as any one of free radical polymerization, condensation polymerization, anionic or cationic polymerization, or step growth polymerization.

The term "cross-linking agent" refers to an agent which induces cross-linking. The cross-linking agent can be any agent that is capable of inducing a chemical bond between adjacent polymeric chains. For example, the cross-linking agent can be a chemical compound. Examples of chemical compounds that can act as cross-linking agent include, but are not limited to, 1-ethyl-3-[3-dimethylaminopropyl]carbodiimide hydrochloride (EDC), vinylamine, 2-aminoethyl methacrylate, 3-aminopropyl methacrylamide, ethylene diamine, ethylene glycol dimethacrylate, methymethacrylate, N,N'-methylene-bisacrylamide, N,N'-methylene-bis-methacrylamide, diallyltartardiamide, allyl(meth)acrylate, lower alkylene glycol di(meth)acrylate, poly lower alkylene glycol di(meth)acrylate, lower alkylene di(meth)acrylate, divinyl ether, divinyl sulfone, di- or trivinylbenzene, trimethylolpropane tri(meth)acrylate, pentaerythritol tetra(meth)acrylate, bisphenol A di(meth)acrylate, methylenebis(meth)acrylamide, triallyl phthalate, diallyl phthalate, transglutaminase or mixtures thereof.

In some embodiments, the hydrogel precursors may themselves be used as cross-linking agents, and do not require addition or use of a separate cross-linking agent.

In some embodiments, the cross-linking agent can be in the form of an electromagnetic wave. Therefore, cross-linking can be carried out using an electromagnetic wave, such as gamma or ultraviolet radiation, which can cause the polymeric chains to cross-link and form a three-dimensional matrix, thereby entrapping water molecules to form a hydrogel. Therefore, choice of cross-linking agent is dependent on the type of polymeric chain and functional group present, and a person skilled in the art would be able to choose the appropriate type of cross-linking agent accordingly.

Cross-linking induced by cross-linking agents can be used to vary the degradation time of the hydrogel used for the composition of the present invention. The hydrogel degrades at a slower rate than the degradable and surface functionalized particle. For example, if gelatin is used as hydrogel as well as for the manufacture of degradable particles, the gelatin used for the hydrogel needs to be modified so that it degrades at a slower rate as the gelatin used for the particles. For this purpose the aforementioned cross-linking agents can be used to vary the degradation time of the hydrogel. The final degradation rate of hydrogel as well as particles can be measured using techniques known in the art. Most commonly the degradation rate of a hydrogel or particle is determined by measuring dry weight loss of the hydrogel or particle over time.

By using one or more of the above mentioned hydrogel precursors, the composition according to the present invention can comprise a hydrogel, which can be formed using one or more of the above mentioned hydrogel precursors. The hydrogel can be a natural hydrogel or a synthetic hydrogel, depending on the type of hydrogel precursor used. Examples of natural hydrogels which are well known in the art include alginate and agarose. In some embodiments of the invention, alginate can be used as the hydrogel. The term "alginate" refers to any of the conventional salts of algin, which is a polysaccharide of marine algae, and which can be polymerized to form a matrix for use in drug delivery and in tissue engineering due to its biocompatibility, low toxicity, relatively low cost, and simple gelation with divalent cations such as calcium ions ($Ca^{2+}$) and magnesium ions ($Mg^{2+}$). Examples of alginate include sodium alginate which is water soluble, and calcium alginate which is insoluble in water. In some embodiments of the invention, agarose can be used as the hydrogel. Agarose refers to a neutral gelling fraction of a polysaccharide complex extracted from the agarocytes of algae such as a Rhodophyceae. However, unlike alginate, it forms thermally reversible gels.

Examples of synthetic hydrogel include, but are not limited to, hydrogels formed from biodegradable polymers, and hydrogels formed from polymers such as polyaminoacids, polyethylene glycol and its derivatives, acrylamide polymers, acrylates polymers, carboxy alkyl celluloses, collagen-hydroxyethylmethacrylate (HEMA), poly(hydroxylethyl methacrylate) (PHEMA), polyphosphazines, polyphosphate esters, polyethylene oxide, polyvinyl alcohol, polyvinylpyrrolidone, polyethyloxazoline, polyethylene oxide-co-polypropyleneoxide block copolymers, PGA-PEG-PGA block copolymers and PGA-PEG diblock copolymers.

Examples of biodegradable polymers include, but are not limited to, polymers and oligomers of glycolide, lactide, polylactic acid, polyesters of a-hydroxy acids, including lactic acid and glycolic acid, such as the poly(a-hydroxy) acids including polyglycolic acid, poly-DL-lactic, poly-L-lactic acid, and terpolymers of DL-lactide and glycolide; e-caprolactone and e-caprolactone copolymerized with polyesters; polylactones and polycaprolactones including poly(e-caprolactone), poly(8-valerolactone) and poly (gamma-butyrolactone); polyanhydrides; polyorthoesters; other hydroxy acids; polydioxanone; and other biologically degradable polymers that are non-toxic or are present as metabolites in the body. Examples of polyaminoacids include, but are not limited to, polylysine (PLL), poly L-aspartic acid, poly L-glutamic acid, and styrene-maleic acid anhydride copolymer. Examples of derivatives of polyethylene glycol includes, but are not limited to, poly(ethylene glycol)-di-(ethylphosphatidyl(ethylene glycol)) (PEDGA), poly(ethylene glycol)-co-anhydride, poly(ethylene glycol)co-lactide, poly(ethylene glycol)-co-glycolide and poly (ethylene glycol)-co-orthoester. Examples of acrylamide polymers include, but are not limited to, polyisopropylacrylamide, and polyacrylamide. Examples of acrylate polymers include, but are not limited to, diacrylates such as polyethylene glycol diacrylate (PEGDA), oligoacrylates, methacrylates, dimethacrylates, oligomethoacrylates and PEG-oligoglycolylacrylates. Examples of carboxy alkyl cellulose include, but are not limited to, carboxymethyl cellulose and partially oxidized cellulose. In one embodiment a polyethylene glycol diacrylate (PEGDA) has been used as hydrogel in the composition of the present invention.

The hydrogel of the present invention is degradable. As used herein, the term "degradable" refers to a material having a structure which can decompose to smaller molecules under certain conditions, such as temperature, abrasion, pH, ionic strength, electrical voltage, current effects, radiation and biological means.

Thermal degradation refers to the use of heat to apply to a material such that it decomposes into smaller molecules. Abrasion degradation or physical degradation refers to the application of force or pressure on the material so as to break down the material into smaller components. Chemical degradation refers to use of a chemical reagent which degrades a material such as hydrogel into smaller molecules through effects of pH or ionic strength of the solution, or through chemical reaction with the material. For example, a form of chemical degradation can be hydrolytic degradation, wherein gelatin undergoes hydrolytic degradation in the presence of water, and can form a product called collagen hydrolysate (CH), which can contain peptides with a mean molecular weight of 3-6 kDa.

Electrical degradation refers to use of electrical current and/or voltage to pass through the material such that the material is decomposed. In radiation degradation, electromagnetic waves such as gamma and ultraviolet waves are used to degrade the material. The hydrogel can also be degraded biologically, i.e. it is biodegradable. The term "biodegradable" refers to a substance which can be broken down by microorganisms, or which spontaneously breaks down over a relatively short time (within 2-15 months) when exposed to environmental conditions commonly found in nature. For example, gelatin can be degraded by enzymes which are present in the body.

In some embodiments, degradation of the hydrogel can take place over a time period ranging from a few seconds to a few days or months. The time period required for the hydrogel to degrade can be dependent on a few parameters, for example, constituent of hydrogel, such as type of hydrogel precursor used and water content of the hydrogel, degree of cross-linking, temperature, pH, amount of aqueous medium present, and pressure during gelation. Under physiological conditions, that means in an animal body, degradation is in general about 2 months. This period can be extended by subjecting the hydrogel to a cross-linking agent as described above.

The hydrogel of the present invention can be substantially composed of water. The amount of water in the hydrogel can be at least 50 wt%, or at least 55 wt%, or at least 60 wt%, or at least 65 wt%, or at least 70 wt%, or at least 75%, or at least 80 wt%, or at least 85%, or at least 90%, or at least 95%, wherein weight percentage is expressed as weight of water with respect to total weight of the hydrogel.

The hydrogel of the present invention can also act as a matrix or framework for additional components. Examples of such additional components include particles, cells, biologically active molecules, molecules, monomers, or comonomers, to name a few. The hydrogel support may covalently bind or non-convalently entrap the additional materials.

The composition according to the present invention comprises at least one kind of degradable and surface cross-linked particle in the degradable hydrogel matrix. Generally, any type of particle that can liquify at elevated temperatures, solidify at room temperature and form cavities within the hydrogel matrix can be used. Typically, a degradable and surface cross-linked particle can be formed by using one or more types of degradable particle precursor, and setting or solidifying the one or more types of degradable particle precursor to form a degradable particle. The term "degradable particle precursor" refers to any chemical compound that can be used to make a degradable particle. Examples of degradable particle precursors include, but not limited to, a hydrocarbon such as paraffin wax, an organic compound such as lard, or a polymer such as a hydrogel described herein.

The degradable particle can degrade by any method described herein. In some embodiments, the degradable particle can contain a degradable material that degrades by melting at body temperature. The term "body temperature" as used herein refers to the range of body temperatures expected for a living mammal, being from about 34 °C to about 40 °C, and generally accepted to be about 37 °C for humans.

In some embodiments, a suitable degradable particle can be formed from a material which degrades faster than the degradable hydrogel matrix. Examples of such particle and hydrogel pairing include, but are not limited to, gelatin and agarose, collagen, collagen derivatives, gelatin and alginate, or gelatin and polyethylene glycol diacrylate (PEGDA). Collagen derivatives can include any type of collagen type from collagen I to collagen XXIX. In one embodiment, collagen I, II, III, IV, V, VI, VII or VIII can be used. Collagen derivates can also include collagen that has been chemically modified. Examples of chemically modified collagen includes, but are not limited to, succinylated collagen, methylated collagen, acetylated collagen, thiolated collagen, and carboxylated collagen. In some embodiments, the degradable particle can also be formed using the same material as that used to form the degradable hydrogel. In such cases, the degradable particle can be made to degrade at a faster rate compared to the hydrogel matrix through, for example, application of pressure, radiation or thermal energy. The degradation time can also be extended by use of cross-linking agents as described further below.

Furthermore, the degradable particles used herein are degradable surface cross-linked particles whose surface has been subjected to a cross-linking agent after manufacture of the degradable particle to introduce cross-links at the surface of the degradable particles. This chemical surface functionalization introduces functional group at the surface of the degradable particles which cross-link and thus can influence the degradability of the particle.

Cross-linking agents which can be used for chemical cross-linking of the surface of the particles can include, but are not limited to 1-ethyl-3-[3-dimethylaminopropyl]carbodiimide hydrochloride (EDC), EDC together with glutaraldehyde, vinylamine, 2-aminoethyl methacrylate, 3-aminopropyl methacrylamide, ethylene diamine, ethylene glycol dimethacrylate, methymethacrylate, N,N'-methylene-bisacrylamide, N,N'-methylene-bis-methacrylamide, diallyltartardiamide, allyl(meth)acrylate, lower alkylene glycol di(meth)acrylate, poly lower alkylene glycol di(meth)acrylate, lower alkylene di(meth)acrylate, divinyl ether, divinyl sulfone, di- or trivinylbenzene, trimethylolpropane tri(meth)acrylate, pentaerythritol tetra(meth)acrylate, bisphenol A di(meth)acrylate, methylenebis(meth)acrylamide, triallyl phthalate, diallyl phthalate, transglutaminase or mixtures thereof.

The degree of surface cross-linking can be influenced by varying the time of exposure of the degradable particle to the cross-linking agent, the ratio of cross-linking agent to degradable particle, and by varying the temperature and/or time during exposure of the degradable particle to the cross-linking agent. The temperature can be at least 4°C or between about 4°C to about 30°C or about 4°C. The temperature used depends on the kind of cross-linking agent and the polymer used for manufacturing the particle. For example, gelatin exposed to EDC and/or gluraraldehyde as cross-linking agent is exposed to the cross-linking agent at temperatures about 4°C. The exposure time can be between about at least 6 h or between about 6 h to about 24 h. In one example the exposure time is between about 12 h to 19 h or is about 12 h or 19 h. The weight ratio of cross-linking agent to degradable polymer can be between about 1:1000 to about 1:10, or between about 1:200 to about 1:40.

The term "gelatin" as used herein refers to protein substances derived from collagen. In the context of the present invention, "gelatin" also refers to equivalent substances such as synthetic analogues of gelatin. Generally, gelatin can be classified as alkaline gelatin, acidic gelatin, or enzymatic gelatin. Alkaline gelatin can be obtained from the treatment of collagen with a base such as sodium chloride. Acidic gelatin can be obtained from the treatment of collagen with an acid such as hydrochloric acid. Enzymatic gelatin can be obtained from the treatment of collagen with an enzyme such as hydrolase. As gelatin can be a form of hydrogel, factors that affect degradation behavior of hydrogels as mentioned herein can apply to gelatin as well.

The degradable particle can be in any shape, such as a sphere, a hemispherical, a cube, a prism, a diamond, in the shape of a finished article or an irregular shape, and can be uniformly dispersed in the hydrogel matrix. Typically, the size of the degradable particle can be between about 100 $\mu$m to about 5000 $\mu$m, or between about 100 $\mu$m to about 2500 $\mu$m, or between about 100 $\mu$m to about 1500 $\mu$m or between about 100 $\mu$m to about 800 $\mu$m, or between about 200 $\mu$m to about 600 $\mu$m, or between about 200 $\mu$m to about 400 $\mu$m. Since the dimensions of a particle is not always regular, i.e. perfectly spherical, the above size refers to the maximal dimension of the particle in any direction. Smaller sizes for the particles are generally more stable than larger particles and have a lower tendency to aggregate.

The weight ratio of degradable hydrogel and degradable particle in the composition can be of any ratio, such as between about 0.01 to 1, or about 0.25 to 1, or about 0.5 to 1, or about 0.75 to 1, or about 1 to 1.

The degradable surface cross-linked particles comprises reactive groups at the surface that allow binding of other molecules on its surface. Examples of such reactive groups include functional groups described herein, and may further include other moieties, such as biotin, avidin, streptavidin, digoxigenin, and anti-digoxigenin. The term "binding" can mean physical bonding or chemical bonding. Examples of other molecules include, but are not limited to, cells, biologically active molecules, particles, molecules, to name a few. In some embodiments, the degradable particle can have reactive groups that allow binding of cells on its surface. Therefore, in such embodiments, the cells which are bound can be present at the periphery of the degradable particles so that they can proliferate and grow at the cavity formed by the degrading particle, which can shorten the time at which tissues are formed since cells do not have to migrate through the hydrogel bulk to the cavity.

The degradable particle can also cross-link with another degradable particle. By the cross-linking of degradable particles, rate of degradation of particles can be controlled. The cross-linking can be carried out by the degradable particles or can be effected via addition of a cross-linking agent described above for degradable particles. In some embodiments, degradation of the degradable particle can take place over a time period ranging from a few minutes to a few days. The time period required for the degradable particle to degrade can be dependent on different parameters, for example, constituent of degradable particle, such as type of degradable particle precursor used and water content of the degradable particle, degree of cross-linking, temperature, pH, amount of aqueous medium present, amount of pressure exerted by the hydrogel matrix on the degradable particle, and pressure during gelation.

In general, degradation of the components of the composition is adapted so that the degradable hydrogel degrades at a slower rate than the degradable surface cross-linked particle. For example, in one embodiment, while hydrogels degrade over a period of 2 months, the degradable surface cross-linked particles, such as surface cross-linked gelatin microspheres, degrade over a period of 1 week. In another example, in which the composition of the present invention is injected at the site of interest to form a degradable scaffold, gelation of the hydrogel after injection takes place in about 1 to 5 min. The degradable surface cross-linked particle injected together with the hydrogel can degrade within one week. Cells, such as mammalian cells injected together with the hydrogel and the degradable surface cross-linked particles start to grow and penetrate into the cavities formed by the degradable surface cross-linked particles after one week in the polymerized hydrogel matrix after 2 to 3 weeks. As shown in the experimental section in one example, tissue islet expansion can take place between about 4 to 6 weeks. At the time the hydrogel is completely degraded after about 2 months, the newly formed cell tissue replaces has formed its own extracellular matrix which takes over the function of the previous hydrogel matrix. The cavities formed by the degraded surface cross-linked particles ensure a homogeneous cell proliferation within the whole hydrogel matrix and not only at its boundaries.

Thus, after injection of the composition of the present invention the following steps occur in the following order: (a) hydrogel matrix formation, (b) surface cross-linked particle degradation to form cavities in hydrogel matrix, (c) cell proliferation and migration within the hydrogel matrix and into the cavities; (d) degradation of hydrogel matrix and tissue islet expansion. With tissue islet expansion it is referred to cells which form on their own a extracellular matrix which replaces the hydrogel matrix.

Degradable particle can in general be obtained by a double-emulsion technique which is known in the art. As already indicated by the word "emulsion", the particles are obtained by emulsification. Emulsification refers to the process of emulsifying in which two immiscible liquids are mixed together (e.g. by shaking, stirring or homogenizing) forming an emulsion. Thus, an emulsion is a disperse system of two or more immiscible liquids. One of the liquids forms a dispersant (also called continuous phase) in which the other phase (also called dispersed phase) is distributed in form of fine droplets. Emulsifying of one liquid in the other results in the formation of small droplets of one liquid dispersed (separated and distributed throughout the space) in the other liquid.

Most emulsions consist of water and oil or fat as immiscible phases. Depending on the composition and ratio of the phases two distribution options exist. In case the aqueous phase, such as water "W" is the continuous phase and the oil "O" is the dispersed phase, the result is an "O/W emulsion" whose basic character is determined by the aqueous phase. If oil "O" is the continuous phase and water "W" the dispersed phase, the result is a "W/O emulsion", wherein the basic character is determined by the oil.

A double-emulsion technique or W/O/W-double emulsion technique is also designated as in-water or in aqueous solution drying method. In general, for this method a two stage emulsification process is used to generate transient $W_1/O/W_2$ emulsions. The principle of particle or microsphere formation is based upon inducing phase separation of the polymer dissolved in an organic solvent (O phase) due to partial extraction of the solvent in a large volume of an external water phase ($W_2$ phase) and evaporation of the volatile solvent. The polymer then forms a coacervate enclosing the internal aqueous phase ($W_1$). Particles or microspheres can then be hardened under removal of residual solvent. Solvents suitable for this technique are those that are good solvents for the polymer, volatile, and immiscible with water. A limited solubility of the solvent in the external water phase can be beneficial.

Another variant of a double-emulsion technique is $O_1/W/O_2$ double emulsion. In this variant an $O_1/W$ emulsion is added to another organic phase ($O_2$). For example, in one embodiment one oil phase (O1), such as ethyl acetate is mixed with a degradable polymer, such as gelatin, that is dissolved in water (W). The resulting emulsion $O_1/W$ is mixed with another oil phase ($O_2$), such as soybean oil. The resulting $O_1/W/O_2$ emulsion is maintained in a cold water bath, and ice cold ethanol can be added to the emulsion for cooling purposes. The microspheres formed were then washed with alternating dioxane/acetone/ethanol solutions to remove soyabean oil before drying.

Such emulsion techniques are different from other know methods of manufacturing particles or granules. For example in one method not used herein for the manufacture of particles, particles, such as gelatin particles are manufactured by autoclaving the polymer solution to sterilize the polymer. If using gelatin for this method, the gelatin is directly obtained from mammalian tissue, such as porcine skin gelatin. After autoclaving the polymer solution is cooled down for gelation. To form the particles or granules the cooling polymer solution is vigorously shaked or stirred to form the particles. The functionality of particles obtained with such mechanical methods is much less controllable.

In contrast, the double-emulsifying method allows to the manufacture of size customized, uniformly shaped microspheres with retarded dissolution property. Also, particles or microspheres obtained with the double-emulsifying method can be subsequently subjected to a cross-linking treatment as described above to obtain degradable surface cross-linked particles or microspheres.

The composition according to the present invention further comprises one or more species of living cells. The term "living cell" refers to any cell that is capable of cell division or contains a nucleus. A "living cell" also refers to a cell that has active metabolic machinery (e.g. mitochondria). The living cells can be eukaryotic cells, prokaryotic cells or archaea. As used herein, the term "eukaryotic cell" refers to any animal or plant cell having a definitive nucleus. Eukaryotic cells of animals include cells of vertebrates such as mammals, and cells of invertebrates such as insects. Examples of eukaryotic cells of plants include yeast cells, and algae cells. Eukaryotic cells can also comprise antibody producing cells, such as hybridoma. The term "prokaryotic cell" refers to a cell of a prokaryotic organism that lacks a definitive nucleus. Examples of prokaryotic cells can include, but are not limited to, the genus Escherichia, Bacillus or Lactococcus. Some examples of prokaryotic cell species from these genera are *Escherichia coli, Bacillus subtilis* or *Lactococcus lactis.* The term "archaea" refers to a group of single-celled microorganisms which has no cell nucleus or any other organelles within their cells.

The eukaryotic cell can be an anchorage dependent cell. An anchorage dependent cell refers to any cell which will grow and multiply when attached to a solid support material, and are not able to grow when present in a suspension. In some embodiments, the anchorage dependent cell can be a mammalian cell. A mammalian cell is any cell that is derived from a mammal. A mammalian cell can include a mammalian cell line. In one embodiment, the mammalian cell can be a human cell. Examples of a human cell include, but are not limited to, an osteogenic cell, a fibroblast, an epidermal cell, an adipocyte, a neural cell, an endothelial cell, an epithelial cell, a keratinocyte, a hepatocyte, , a myocyte, a cell from joint ligament, a cell from the nucleus pulposis, a HEK 293 cell and PER.C6® cell. For such cells, the conditions of attachment of the cells to a particular substrate greatly influence their subsequent function.

The composition referred to herein is particularly suitable for the use with non-anchorage dependent cells. Non-anchorage dependent cells can be further classified into Type A and Type B. Type A non-anchorage dependent cells refer to cells that are able to grow and multiply in the absence of a solid support material. For example, Type A non-anchorage dependent cells are able to proliferate in a suspension. Examples of Type A non-anchorage dependent cells include carcinoma cells used for regenerative medicine. Carcinoma cells used for regenerative medicine can include, but are not limited to hepato-carcinoma cells or pancreatic carcinoma cells. Type B non-anchorage dependent cells refer to cells that can grow and multiply when attached to a solid support material, and are also able to grow and multiply in the absence of a solid support material. Examples of Type B non- anchorage dependent cells include, but are not limited to chondrocytes, embryonic stem cells, adult stem cells, and endodermal lineage cells. For example, chondrocytes are able to proliferate in hydrogels, which are not considered as solid support materials. In the absence of adhesive moieties in the hydrogels, chondrocytes can adopt favorable spherical phenotype and undergo normal proliferation.

The term "chondrocyte" refers to a cell that is capable of expressing characteristic biochemical markers of chondrocytes such as, but not limited to collagen type II, chondroitin sulfate, and keratin sulfate, and is able to generate tissue or matrices with hemodynamic properties of cartilage *in vitro.* An osteogenic cell refers to an osteoblast or a progenitor osteoblast cell, which gives rise to a bone tissue. A fibroblast is a spindle shaped cell which can rapidly replicate and synthesize a fibrous matrix composed of a variety of extracellular matrix molecules including Type I Collagen, and which can be found in skin. An epidermal cell refers to a cell of the epidermis, wherein the epidermis is the outer layer of skin and is composed of four types of cells, i.e. keratinocyte, melanocyte, Langerhans cell, and Merkel cell. The term "adipocyte" refers to a cell existing in or derived from fat tissue which is terminally differentiated. It is also known as a lipocyte or fat cell, and specializes in storing energy as fat. In their differentiated state, adipocytes assume a rounded morphology associated with cytoskeletal changes and loss of mobility. Neural cells refer to cells of the nervous system and in particular of the brain. Examples of neural cells include, but are not limited to, neurones, astrocytes and oligodendrocytes. Endothelial cells refer to a thin, flattened cell, of which a layer of the cells lines the inside surfaces of body cavities, blood vessels and lymph vessels, making up the endothelium. The term "epithelial cell" refers to a cuboidal-shaped, nucleated cell which is generally located on the surface of a tissue. A layer of epithelial cells generally functions to provide a protective lining and/or surface that may also be involved in transport processes. The term "keratinocyte" refers to skin cells having the capability to produce keratin, including for example, cells known as basal cells, prickle cells, spinous cells, and granular cells. A hepatocyte is a cell that constitutes the main functional cells of the liver, and can constitute 60 to 80% of the mass of a liver tissue. Hepatocytes perform critical metabolic, endocrine, and secretory functions, which includes the synthesis of carbohydrates, cholesterol and bile salts, to name a few. Stem cells refer to cells having self-replicating ability and also the ability to differentiate into at least two cells, and can be divided into pluripotent stem cells and multipotent stem cells. Myocte refers to a differentiated, post-mitotic, muscle cell that has not undergone fusion and represents a transient cell type under most conditions. Cell from joint ligament can comprise a chondrocyte or a fibroblast from the articular ligament, peritoneal ligament or fetal remnant ligant, which are important as ligaments connect a bone to another bone to form a joint which is required for mobility. Cells from the nucleus pulposis have chondrocyte-like features. In an adult human, the cells of the nucleus pulposis obtain nutrients and eliminate waste by diffusion through blood vessels in the endplates of the vertebrate adjacent to the intervertebral discs. A HEK 293 cell is a human embryonic kidney cell line, and PER.C6® cell is a human retina cell line.

In some embodiments, the one or more species of living cells mixed together with the at least one degradable hydrogel and the at least one degradable particle. The hydrogel can be in the form of its precursor. In some embodiments, the at least one degradable hydrogel and the at least one degradable particle can be mixed together before the one or more species of living cells are added. In some embodiments, the living cell can be provided with the degradable particle precursor, such that the degradable particle precursor can form a degradable particle comprising the living cell.

In some embodiments, the composition can be poured or injected into a mold having a desired anatomical shape, and then hardened to form a matrix having cells dispersed therein, which can be transplanted into a patient. The hydrogel can degrade, leaving only the resulting tissue. In some embodiments, the composition is adapted to be deliverable to a site, such as a defect site, in an animal or a human body. The composition can be injected directly into a site, such as a defect site, in a patient, where the hydrogel can harden into a matrix having cells dispersed therein. The hydrogels are also biocompatible, e.g., not toxic, to cells suspended in the hydrogel. It is also possible to let the hydrogel form and afterwards shape the composition to match the size and shape of a defect site in which it is to be implanted.

The composition according to the present invention can also serve as a cell growth medium. Cells can be introduced into a composition comprising a degradable hydrogel and a degradable particle as described herein to produce a cell construct. The cell construct is incubated under conditions suitable for growth of the cells. That is, the cell construct can be placed in an incubator or into a patient so that the cells are maintained under adequate environmental conditions to permit the cells to survive, proliferate, differentiate and/or express certain products. "Cell growth" means that the cells survive and preferably, though not exclusively, divide and multiply. The composition can be adapted to induce tissue generation. In some embodiments, the composition may comprise cell growth media, which typically provides necessary nutrients and environmental conditions for cell growth. The cells may be introduced and incubated under conditions suitable for cell growth by introducing the composition into a patient and allowing native cells, such as stem cells to migrate into the composition. The composition can be administered by injecting the composition into the region requiring cellular growth or remodeling, such as a region of damaged tissue or a defect site.

In some embodiments, the composition according to the present invention can include a biologically active molecule. As used herein, "biologically active molecules" are defined as those organic molecules having an effect in a biological system, whether such system is *in vitro, in vivo,* or *in situ.* Biologically active molecules can include, but are not limited to growth factors, cytokines, antiseptics, antibiotics, anti-inflammatory agents, analgesics, anesthetics, chemotherapeutic agents, clotting agents, metabolites, chemoattractants, hormones, steroids, and other drugs, or cell attachment molecules.

The term "growth factors" refers to factors affecting the function of cells such as osteogenic cells, fibroblasts, neural cells, endothelial cells, epithelial cells, keratinocytes, chondrocytes, myocytes, cells from joint ligaments, and cells from the nucleus pulposis. Platelet derived growth factors (PDGF), the transforming growth factors (TGF-.beta.), insulin-like growth factors (IGFs), fibroblast growth factors (FGFs), and the bone morphogenetic proteins (BMPs) are examples of growth factors encompassed in the composition according to the present invention. The term "cytokines" refers to peptide protein mediators that are produced by immune cells to modulate cellular functions. Examples of cytokines include, but are not limited to, interleukin-1$\beta$ (IL-1$\beta$), interleukin-6 (IL-6), and tumor necrosis factor-$\alpha$ (TNF$\alpha$).

The term "antiseptics" refers to a chemical agent that inhibits growth of disease-carrying microorganisms. Examples of antiseptics include peroxides, C6-C14 alkyl carboxylic acids and alkyl ester carboxylic acids, antimicrobial natural oils, antimicrobial metals and metal salts such as silver, copper, zinc and their salts. The term "antibiotic" includes bactericidal, fungicidal, and infection-preventing drugs which are substantially water-soluble such as, for example, gentamicin, vancomycin, penicillin, and cephalosporins. An antibiotic can be added, for example, for selection of the cells or to prevent bacterial growth in the composition. The term "anti-inflammatory agent" refers to any agent possessing the ability to reduce or eliminate cerebral edema (fluid accumulation) or cerebral ischemia, and can include examples such as free radical scavengers and antioxidants, non steroidal anti-inflammatory drugs, steroidal anti-inflammatory agents, stress proteins, or NMDA antagoists. The term "analgesics" refer to drugs which eliminate or alleviate pain without losing consciousness. Analgesics are generally classified, for example, into narcotic analgesics such as morphine, non-narcotic analgesics such as aspirin, and narcotic antagonistic analgesics which develop analgesic action through a mechanism similar to that of narcotic analgesics. The term "anesthetics" refers to an agent that produces a reversible loss of sensation in an area of a subject's body. Examples of anesthetics include bupivacaine, levobupivacaine, lidocaine, prilocalne, and cocaine.

The term "chemotherapeutic agents" refer to any natural or synthetic molecules that are effective against one or more forms of cancer, and can include molecules that are cytotoxic (anti-cancer agent), simulate the immune system (immune stimulator) or molecules that modulate or inhibit angiogenesis. Examples of chemotherapeutic agents include alkylating agents, antimetabolites, taxanesm, cytotoxics and cytoprotectant adjuvants. The term "clotting agent" refers to refers to any molecule or compound that promotes the clotting of blood. Examples of clotting agents include a thrombin agent, which is commonly used as a topical treatment by vascular surgeons to stop surface bleeding after a large surface incision is made in the body, heparin, warfarin, and coumarin derivatives. The term "metabolite" refers to an intermediate or a product derived from enzymatic conversion of a substrate administered to a subject, the conversion occurring as part of a metabolic process of the subject. Examples of metabolite include glucose, carbohydrates, amino acids and lipids. The term "chemoattractants" refers to a substance that elicits accumulation of cells, such as chemokines, monocyte chemoattractant protein-1, and galectin-3. The term "hormone" refers to trace substances produced by various endocrine glands which serve as chemical messengers carried by the blood to various target organs, where they regulate a variety of physiological and metabolic activities in vertebrates. Examples of hormones include steroidal estrogens, progestins, androgens, and the progestational hormone progesterone. Steroids can also be classified as lipids. Naturally occurring steroids are hormones that are important regulators of animal development and metabolism at very low concentrations. Examples of steroids include cholesterol, cortisone, and derivatives of estrogens and progesterones. The term "cell attachment molecules" as used herein includes, but is not limited to, fibronectin, vitronectin, collagen type I, osteopontin, bone sialoprotein thrombospondin, and fibrinogen. Such molecules are important in the attachment of anchorage-dependent cells.

In a second aspect, the invention provides a method of manufacturing a composition. The method comprises mixing one or more species of living cells, at least one degradable hydrogel and at least one kind of degradable and surface cross-linked particle to form a hydrogel comprising the one or more species of living cells and the at least one kind of degradable and surface cross-linked particle, wherein the at least one kind of degradable and surface cross-linked particle comprises a material which degrades faster than the at least one degradable hydrogel, wherein the at least one kind of degradable and surface cross-linked particle has a surface comprising additional reactive groups to bind the one or more species of living cells to its surface, and wherein the one or more species of living cells bound to the surface of the at least one kind of degradable and surface cross-linked particle are comprised in the at least one degradable hydrogel, and wherein the at least one degradable hydrogel of the mixture is formable to a hydrogel matrix.

Using an optical microscope, the inventors have observed that there is a high level of both cell proliferation and extracellular matrix (ECM) production using a hydrogel matrix comprising cavities. The term "extracellular matrix" refers to a complex, three-dimensional network of macromolecules, such as proteins, that provides an architectural scaffold for cellular adhesion and migration and which can also act as a mechanical support and biochemical barrier. It can be made up of amorphous and fibrillar components of tissues and blood including collagen, laminin, fibronectin, vitronectin, their subtypes and combinations thereof. In mammals, the ECM can surround cells as fibrils, tubes and channels that contact the cells on all sides, or as a sheet called the basement membrane that cells 'sit on'. A modified form of ECM for example appears in form of bone.

Furthermore, through carrying out experiments using non-anchorage dependent cells on non-cell adhesive hydrogels, the inventors have made the finding that the non-anchorage dependent cell is able to maintain vigorous viability and proliferative tendency, while being trapped and confined in the framework lattice of non-cell adhesive hydrogel scaffold. The cells can gradually propagate into isolated spherical colonies within the hydrogel bulk and would stop growing after their growth is limited by the physical constraint of the hydrogel. Therefore, the cavities in the hydrogel matrix can physically direct and accommodate the growth of neo-tissues inside the cavities. The neo-tissues can eventually fill the cavities, and can ultimately fuse together within the hydrogel matrix.

A method of manufacturing a scaffold for tissue engineering is also disclosed, wherein the method comprises providing a mixture comprising at least one degradable hydrogel, at least one kind of degradable particle and one or more species of living cells to form a hydrogel comprising the at least one kind of degradable particle and the one or more species of living cells. The degradable particle comprises a material which degrades faster than the degradable hydrogel. The method includes incubating the mixture under conditions which allow proliferation of the one or more species of living cells and degradation of the at least one degradable particle in the hydrogel, so as to allow the at least one degradable particle to degrade and to allow the one or more species of living cells to proliferate. The method includes degrading the at least one degradable hydrogel of the incubated mixture to obtain a scaffold for tissue engineering.

As used herein, the term "scaffold" refers to a highly porous, artificial, three-dimensional network of interconnected pores that is used *in vivo* as a framework to which additional cells can attach and both existing and additional cells can grow to regenerate tissues, which can be lost through injury or disease. The term "living scaffold" refers to a scaffold that can be formed from and by living cells. One or more species of living cells can be attached to the scaffold via physical bonding or chemical bonding described herein. The living cells can be allowed to proliferate for a time period, in which the cells can grow to form colonies, after which the colonies can fuse to form a network of cells, and subsequently forming a living scaffold. Generally, the time for proliferation can range from a few hours or days to a few weeks, such as about 1 day to about 4 weeks, or about 1 day to about 2 weeks, or about 1 day to about 1 week. The time for proliferation can also depend on the cultivation conditions for the cells. Parameters of the cultivation condition can include, for example, temperature, pH, amount of water, pressure, nutrients present, and type of cell. For example, it is well known that eukaryotic mammalian cells grow much slower in general than for example prokaryotic bacterial cells. Cultivation conditions of cells are known in the art and can therefore be adapted by a person skilled in the art depending on the desired cell type and application.

The hydrogel matrix can degrade naturally. The term "degrade naturally" refers to subjecting the hydrogel matrix in an environment of intended use such that the hydrogel is degraded. The hydrogel matrix can also be degraded artificially, using one of the degradation modes as described herein. The degradation rate of the hydrogel matrix can be adjusted so as to suit the rate of cell proliferation. For example, the hydrogel matrix can be degraded at a faster rate compared to the rate of cell proliferation, so that the cells can have sufficient space for growth.

In some embodiments, one or more species of living cells can be added after incubating the mixture, wherein the one or more species of living cells added after incubating the mixture comprises the same or different species of cells than the cells already mixed into the mixture together with the at least one degradable hydrogel and the at least one kind of degradable particle. Generally, different living cell species can be added into the mixture depending on the type of tissue or organ for implantation. The term "tissue" refers to a structure formed by related cells joined together, wherein the cells work together to accomplish specific functions. An organ refers to a differentiated structure of an organism composed of various cells or tissues and adapted for a specific function. Therefore, one or more species of living cells can be added into the mixture to form a specific organ. For example, the heart which is an organ contains muscle tissue that contracts to pump blood, fibrous tissue that makes up the heart valves and special cells that maintain the rate and rhythm of heartbeats.

A scaffold according to the present invention can be used for a wide variety of applications, e.g. tissue engineering. It can be used, e.g., for the three dimensional expansion of autologous cells like bone marrow mesenchymal stem cells which are limited due to donor site morbidity. The host for such applications may be any suitable animal. In a further embodiment said host is a mammal or a human patient.

A scaffold according to the present invention can also be used in transplantation as a matrix, for example, dissociated cells such as chondrocytes or hepatocytes to create a three-dimensional tissue or organ. Any type of cell can be added to the scaffold for culturing and possible implantation, including cells of the muscular and skeletal systems, such as chondrocytes, fibroblasts, muscle cells and osteocytes, parenchymal cells such as hepatocytes, pancreatic cells (including Islet cells), cells of intestinal origin, and other cells such as nerve cells and skin cells, either as obtained from donors, from established cell culture lines, or even before or after genetic engineering. Pieces of tissue can also be used, which may provide a number of different cell types in the same structure. The scaffold can also be used as a three dimensional *in vitro* culture system for attachment-dependent cells, e.g., hepatocytes in a three dimensional microenvironment which mimics the physiological microenvironment more closely.

The invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non- limiting examples. In addition, where features or aspects of the invention are described in terms of Markush groups, those skilled in the art will recognize that the invention is also thereby described in terms of any individual member or subgroup of members of the Markush group.

## EXPERIMENTAL SECTION

Figure 1A, 1B and 1D show a general scheme of the method of manufacturing a composition, otherwise called the "phase transfer cell culture" (PTCC) method. In the embodiment shown in Figure 1A, one or more species of living cells **103** is co-suspended with at least one kind of degradable particles **102** and at least one kind of degradable hydrogel precursor **101'** in an aqueous medium. The composition comprising the living cells **103,** degradable particles **102** and degradable hydrogel precursor **101'** can be set or solidified in the aqueous solution to form a degradable hydrogel matrix **101,** in which the living cells **103** and degradable particles **102** can be encapsulated. As shown Figure 1B, the degradable particles can degrade under certain conditions, forming cavities in the hydrogel matrix. In Figure 1D, row (a) shows how the cells in the hydrogel matrix can proliferate and fill the cavities. With formation of the cavities as a result of degradation of the degradable particles, the cells can proliferate in the hydrogel matrix, followed by sprouting out of the gel-cavity interface, with subsequently formation of neo-tissues against the inner surface of the cavities. The cavity can be filled up by the neo-tissues, forming spherical islets, and subsequent fusing together of the islets.

### Example 1: Surface-crosslinked gelatin microspheres preparation

Gelatin microspheres were fabricated using plain or Rhodamine B (Aldrich) pre-labeled gelatin (Sigma) following a double emulsion method. 10 mL ethyl acetate was mixed with 30 mL gelatin solution (0.1 g/mL in water) by stirring. The emulsion was then added to 60 mL soybean oil and stirred for 10 min. Subsequently, the solution was added to 300 mL pre-cooled (-20 °C) ethanol and rinsed with 1,4-dioxane/acetone, after which gelatin microspheres were formed spontaneously. The gelatin microspheres, which had a diameter of about 150 to about 180 μm, were air dried and were collected via standard sieves (80~100 mesh). Through varying the concentration of gelatin solution, time of reaction and temperature, different sizes of gelatin microspheres can be obtained.

A partial surface-crosslinking treatment was conducted on the sieved microspheres via suspending the microspheres in N-ethyl-N'-(3-dimethylaminopropyl) carbodiimide solution (EDC, in 90% ethanol) at 4 °C for 12 hrs, followed by suspending in phosphate buffered saline (PBS) containing trace amounts of glutaraldehyde which works to improve the thermal resistance, decrease the solubility in water as well as to improve the mechanical properties of gelatin at 4 °C for 19 hrs. Through adjusting the ratio of gelatin versus EDC, use of a different solvent besides ethanol for EDC and varying the time for crosslinking, rate of dissolution of gelatin microspheres can be controlled.

Subsequently, the products were washed in pure ethanol and dried in vacuum oven.

### Example 2: Acellular Micro-cavitary gel (MCG) construction

The partially surface-cross-linked gelatin microspheres were pre-equilibrated by suspending the microspheres overnight in phosphate buffered saline at 4 °C. Subsequently, the microspheres were suspended in agarose solution (0.02 g/mL, in phosphate buffered saline) at a temperature of 37 °C, using a solution to microsphere ratio of 1 mL solution to 0.2 g microsphere. Irrespective of the hydrogel used and the degradable particle material, the ratio of mixing ratio can be between about 1 ml of hydrogel to between about 0.2 to 0.5 g of degradable particle material. After injection into a mold, the mixture was solidified by cooling down to a temperature below 25 °C. By controlling the speed of melting of the encapsulated gelatin microspheres, cavities were created inside the agarose gel bulk.

**Example 3: Chondrocytes isolation and culture**

Chondrocytes were isolated from cartilage tissue of edible porcine articular cartilage. Firstly, cartilage tissue obtained from the joint of a 5-month-old pig was cut into small chips. Chondrocytes were isolated by incubating the cartilage pieces in Dulbecco's Modified Eagle Medium (DMEM) culture medium with 10% (v/v) fetal bovine serum (FBS) containing 1 mg/mL collagenase type II at 37 °C for 12 hrs under gentle stirring.

The chondrocytes were centrifuged, and resuspended in DMEM supplemented with 20% (v/v) FBS, 0.01 M 4-(2-hydroxyethyl)-piperazine-1-ethanesulfonic acid (HEPES), 0.1 mM nonessential amino acids (NEAA), 0.4 mM proline, 0.05 mg/mL vitamin C, 100 units/mL penicillin and 100 mg/mL streptomycin. The cell suspension was then seeded in 75 mL tissue culture flask (Falcon, seeding density $2 \times 10^4$ cells/$cm^2$) and incubated in humidified air with 5% carbon dioxide ($CO_2$) at 37 °C for routine culture. After a confluent cell layer was formed (7-10 days), the chondrocytes (Passage I) were detached using 0.25% (w/v) trypsin in PBS. The hydrogel solution was added to re-suspend the chondrocytes for cell-laden micro-cavitary gel (MCG) construction.

**Example 4: Extraction of "Edge flourish" (EF) layer**

The inventors of the present application have observed that, for a long term culture of cell-laden hydrogel constructs *in vitro,* there is a higher degree of cell growth at the edge of the culture medium compared to the level of cell growth in the bulk of the culture medium. These cell growths at the edge of the culture medium can result in neo-tissue formation forming a scaffold-free phase that covers the entire outer surface of the cell-laden hydrogel construct. The inventors have called this phenomenon the "edge flourish" mechanism.

For better understanding edge flourish (EF) phenomenon and in-depth investigation of the superior performance of edge flourish layer in the conventional hydrogel, cylindrical chondrocyte-laden agarose constructs were prepared.

The chondrocytes were suspended in agarose gel solution (0.02 g/mL, in phosphate buffered saline, cell density $1.2 \times 10^7$ cells/mL) at 37 °C and injected into a mold having a diameter of 4 mm and height of 3 mm. After solidification at room temperature, the cylindrical cell/gel constructs were transferred to a 24-well culture plate, wherein the cells were cultivated at 5% carbon dioxide ($CO_2$) at a temperature of 37 °C for a time range of 3 to 32 days.

In order to separate the edge flourish layer from the gel bulk, surface of the gel construct was removed, forming a hollow cylindrical edge flourish layer and a solid cylindrical gel bulk. The top and bottom surfaces of the solid gel bulk were also cut off as parts of the edge flourish layer. The edge flourish layer and gel bulk were thus distinguished and separated for subsequent measurements/tests.

Figure 5A are optical microscopic images of cell growth illustrating the edge flourish effect. In row (a), a spherical colony of cells is located at the hydrogel-air interface, whereas in row (b), a spherical colony of cells is located at the bulk of the hydrogel. As shown in row (a), the cells located at the hydrogel-air interface are able to sprout out of the gel and form a larger colony of cells compared to that shown in row (b), in which the growth of the cells are constraint.

Figure 7 shows the edge flourish mechanism as an illustration in row (a), and a histological illustration in row (b). The cells can be encapsulated in a hydrogel as shown in Day 3. At Day 12, it was observed that the cells start to migrate outwards towards the outside of the hydrogel, and at Day 40, a layer of neo tissue has been formed on the outer surface of the hydrogel.

**Example 5: Cell-laden micro-cavitary gel (MCG) construction**

Agarose and alginate were used to form cell-laden micro-cavitary gel constructs. Chondrocytes were suspended in an agarose gel solution (0.02 g/mL, in phosphate buffered saline, cell density $1.2 \times 10^7$ cells/mL) and an alginate gel solution (0.012 g/mL, in phosphate buffered saline, cell density $1.2 \times 10^7$ cells/mL), and separately mixed with surface-crosslinked gelatin microspheres to form micro-cavitary gel constructs.

For agarose, the micro-cavitary gel constructs were solidified using a method outlined in Example 2 above. For alginate, the micro-cavitary gel constructs were formed by dispensing the alginate-cell suspension drop-wise (40 $\mu$L/drop) into a 102 mM calcium chloride solution using pipette.

The final cell densities of agarose and alginate constructs were both about $1 \times 10^7$ cells/mL. As the control, conventional hydrogel constructs were prepared by encapsulating chondrocytes (cell density $1 \times 10^7$ cells/mL). The constructs were cultivated in cell culture medium (CCM) with shaking (60 rpm) in the cell culture incubator (5% $CO_2$, 37 °C) for up to 32 days. The culture medium was changed every 2-3 days.

The agarose micro-cavitary gel constructs were used to evaluate the overall performance of tissue regeneration. Because the cells embedded in the alginate can be re-isolated without any harm using an alginate recovered chondrocytes (ARC) method, such as that exemplified in K. Masuda et al, J. Orthop. Res., 2003, 21, 139, the alginate micro-cavitary gel constructs were employed to analysis the gene expression in details.

Figure 1C are optical microscopic images showing the formation of cavities in a hydrogel matrix. In this embodiment, living cells of chondrocytes are stained with fluorescent calcein illuminating in green, and gelatin particles are pre-labeled with Rhodamine B. From the images, it can be seen that the red luminance associated with the gelatin particles gradually diminishes and is almost invisible after 2 days. Insert SEM image of Figure 1C(iii) confirms that cavities have been formed in place of the gelatin particles, which show up as dark blank vacancies in the SEM image.

Figure 2A are optical microscopic images showing chondrocytes culture in alginate hydrogel with phase transfer cell culture (PTCC) structure for a time period ranging from 3 to 42 days. As shown in the images, the chondrocytes aggregated and formed cell colonies around the cavities, after which the cells continue to sprout out from the gel-cavity interface and continue to grow into the cavities. At Day 42, the cavities were almost filled up by the cells.

Figure 2B are optical microscopic images showing chondrocytes culture in polyethylene glycol diacrylate (PEGDA) hydrogel with phase transfer cell culture PTCC structure after a culture time period of 28 days. The image shows that cell colony was formed which filled the cavity. Similar to using agarose with PTCC structure, cells, spheres and PEGDA precursor solution with photo-initiator were mixed well and then put under UV light for polymerizing to form gels which were then used for the experiments described herein.

## Example 6: Formation of cavities of different sizes

Phase transfer cell culture (PTCC) method has been performed in the hydrogel with different cavities sizes from 200 μm to 5mm. Figure 3 shows optical microscopic images showing effects of cavity size on cell colony formation. The results show that typical PTCC process including cell colony formation and invading of the cavities by the cells has occurred.

## Example 7: Cell viability and scanning electron microscopy observation

"Live/Dead" dye staining (Molecular probes, Invitrogen Singapore) was used to determine the viability of chondrocytes. The loaded viable cells were indicated with green fluorescent calcein. Field emission scanning electron microscopy (FESEM, JSM-6700F, JEOL Ltd., Tokyo, Japan) was used to examine the microstructure of micro-cavitary gel constructs.

The glutaraldehyde (2.5%)-preserved micro-cavitary gel constructs were treated with 1% osmium tetroxide ($OsO_4$) at 4 °C for 2 hrs. Subsequently, the constructs were dehydrated using gradient ethanol solutions and further dried in vacuum overnight. Platinum was sputtered for scanning electron microscopy (SEM) contrast.

## Example 8: Agarose content in micro-cavitary gel constructs determination

In order to trace the fate of hydrogel component in phase transfer cell cultural (PTCC) system quantifiably, the agarose content of lyophilized cell-laden micro-cavitary gel constructs was measured by determining 3,6-anhydrogalactose content roughly using a method well known in the art (exemplified in Yaphe et al Anal. Biochem., 1965, 13, 143), compared to that of the constructs without cavities.

Using pure agarose as standard, the residual agarose content at given time points was calculated according to the following equation:

$$\textit{Agarose remaining percentage \%} = W_t / W_0$$

wherein $W_0$ and $W_t$ denote the weight of agarose at day 0 and other given time point respectively.

## Example 9: Cell islets isolation from alginate micro-cavitary gel constructs

The alginate recovered chondrocyte (ARC) method was used to distinguish the cell islets induced by phase transfer cell cultural (PTCC) system from individual cells in the gel-bulk. When the obvious cell aggregation could be found in optical microscopy, the alginate micro-cavitary gel constructs were collected and dissolved in a buffer containing 55 mM sodium citrate, 0.15 M sodium chloride, pH 6.8, for 10 min at 4 °C, in order to remove alginate and thus release scattered individual or aggregated cells.

The resulting suspension of chondrocytes (mixture individual and aggregated cells) was filtered by a cell strainer (40 μm Nylon; BD Falcon, Bedford, MA), wherein the aggregated chondrocytes on the strainer and the scattered individual ones below the strainer were collected separately. The two kinds of cells were centrifuged and subsequently washed and resuspended in phosphate buffered saline so as to remove residual sodium citrate.

**Example 10: Biochemical and (immuno-) histological evaluation**

Biochemical assays were performed on the lyophilized cell-laden micro-cavitary gel constructs. The specimens were digested with 1 mL papain solution (0.3 mg/mL, mixed with 0.1 mM disodium ethylenediaminetetraacetic acid in 0.2 mM dithiothreitol) for 24 hours. By centrifugation, the supernatant containing digested cell lysate was collected for measurement of cell numbers (via DNA quantification using Hoechst 33258 dye),5-6 collagen content (via hydroxyproline quantification), and glycosaminoglycan (GAG) content (with Dimethylmethylene blue).

For histological evaluation, paraformaldehyde (4%) -preserved sample slices were respectively stained with hematoxylin and eosi (H&E), Safranin O and Masson's trichrome dyes. Collagen II primary antibody (2 $\mu$g/mL in PBS, MAB8887, Chemicon) and Anti-IgG (5 $\mu$g/mL in PBS, InvitrogenAlexaFluor, 488) were utilized for collagen II staining and NCAM antibody with fluorescein conjugates (sc-7326 FITC, Santa Cruz Biotechnology) was applied for NCAM staining.

**Example 11: Real time polymerase chain reaction (RT-PCR)**

In order to analyze the gene expression level of cells derived from various experimental samples, RT-PCR was conducted via the following procedures. All the RT-PCR reagents used were purchased from Promega (Madison, MI, USA). **Table 1** lists the primers used, and all the oligonucleotides were synthesised by AIT Biotech (Singapore).

**Table 1** List of PCR primers used

| Genes | Primer sequence (both 5' - 3') (F and R stand for forward and reverse sequence respectively) |
|---|---|
| GAPDH | F: ACCCCTTCATTGACCTCCAC (SEQ ID NO: 1); R: ATACTCAGCACCAGCATCGC (SEQ ID NO: 2) |
| HPRT1 | F: GGACTTGAATCATGTTTGTG (SEQ ID NO: 3); R: CAGATGTTTCCAAACTCAAC (SEQ ID NO: 4) |
| RPL4 | F: CAAGAGTAACTACAACCTTC (SEQ ID NO: 5); R: GAACTCTACGATGAATCTTC (SEQ ID NO: 6) |
| TBP1 | F: AACAGTTCAGTAGTTATGAGCCAGA (SEQ ID NO: 7); R: AGATGTTCTCAAACGCTTCG (SEQ ID NO: 8) |
| Collagen II | F: GCTATGGAGATGACAACCTGGCTC (SEQ ID NO: 9); R: CACTTACCGGTGTGTTTCGTGCAG (SEQ ID NO: 10) |
| Aggrecan | F: CGAGGAGCAGGAGTTTGTCAAC (SEQ ID NO: 11); R: ATCATCACCACGCAGTCCTCTC (SEQ ID NO: 12) |
| RhoA | F: AGCTGGGCAGGAAGATTATG (SEQ ID NO: 13); R: TGTGCTCATCATTCCGAAGA (SEQ ID NO: 14) |
| Integrin $\beta$1 | F: TGCCAAATCATGTGGAGAATGTAT (SEQ ID NO: 15); R: GTCTGTGGCTCCCCTGATCTTA (SEQ ID NO: 16) |
| Sox9 | F: GCTGGCGGATCAGTACCC (SEQ ID NO: 17); R: CGCGGCTGGTACTTGTAA (SEQ ID NO: 18) |
| COMP | F: GGCACATTCCACGTGAACA (SEQ ID NO: 19); R: GGTTTGCCTGCCAGTATGTC (SEQ ID NO: 20) |

RNA from specimens was extracted using the combination of TRIzol® (Invitrogen) and RNeasy® Mini Plant Kit (Qiagen, Düsseldorf, Germany). The acquired RNA samples (from cell-laden constructs) were treated with RNase-free DNase I and converted to cDNA for subsequent PCR experiments. For real-time quantitative PCR, the relative gene expression values were obtained from iQ™ qPCR system (Bio-Rad, Hercules, CA, USA) and calculated with the comparative $\Delta\Delta$CT (threshold cycle) method, as normalized to the housekeeping gene.

**Example 12: Animal Experimentation**

All the animal experiments were performed under guidelines approved by the Institutional Animal Care and Use Committees (IACUC), SingHealth, Singapore. Specimens with chondrocytes (agarose model) were implanted subcutaneously in the dorsum of athymic nude mice after 7 to about 10 days culture *in vitro.* The nude mice were anesthetized by Ketamine (40 mg/Kg) and Diazepam (5 mg/Kg) mixture and disinfected with 70% ethanol and iodine. Two incisions were made on the back skin of each animal, and the specimens were implanted subcutaneously through each of the incisions. After suturing, the animals were raised for 3 or 6 weeks and then euthanized by cervical dislocation after anesthetizing the mice. The embedded constructs were taken out from the subcutaneous tissue for bio-analysis.

Animal experiment data show that the ECM secretion in the cavity sites was higher than the other sites and control as expected, which indicated that the PTCC construct also has good performance *in vivo.* Figure 4 shows optical microscopic images of histological staining of hydrogel implanted subcutaneously in nude mice for 1 week. The images show that the cavities in PTCC constructs were filled up with ECM. The collagen density was much higher than that in the control (normal hydrogel without cavities formed by degraded particles) (Masson staining).

*In vivo* trials were also conducted via subcutaneous implantation of MCG constructs in nude mice, from which flourishing and bona fide cartilaginous neo-tissues are yielded after a few weeks of cultivation. Figure 6C shows the optical microscopic images of histological staining of the hydrogel implanted subcutaneously in nude mice.

## Example 13: Statistical Analysis

Biochemical data from cell-laden hydrogels constructs with cavities were compared to that obtained from cell-laden hydrogel constructs without cavities with equal incubation times using student's t-test, with 3 specimens in each group. The significant level was set as $p < 0.05$. Results are reported as mean $\pm$ standard deviation.

Figure 5B and 5C are graphs showing phenotypic evidences on both transcriptome and proteomic level of the mechanism for edge flourish phenomenon. From RT-PCR, the level of gene expression can be compared and further the capability of cell proliferation and secretion can also be compared. As the gene expressions in surface layer are higher, it can be concluded that cells in surface layer performed better than those in hydrogel bulk.

Positive expression of cellular connective proteins such as N-CAM (as seen in Figure 5A), COMP, and integrin strongly indicates the formation of colonies and their subsequent role as the basic units of all substantial actions taken, including developmental expansion and trans-phase outgrowth, which further implies their cellular state virtually free of focal adhesion. The observably greater expression of RhoA in surface layer reflects a higher proliferative profile of the local cell population that ultimately constitutes the EF per se.

Figure 6A and 6B are graphs demonstrating that the maintenance of chondrocytic phenotype is better in cells residing in agarose hydrogel based MCG-PTCC model. From RT-PCR and biochemistry assay, the level of gene expression can be compared, and further the capability of cell proliferation and ECM secretion amount can also be compared. As the gene expressions and ECM secretion in PTCC sample are higher, it can be concluded that cells in PTCC samples performed better than those in Control samples. Superior gene expression and proteinic yields of the cartilage markers including chondrogenic transcription factor (Sox9), cartilaginous collagen (type II) and proteoglycan (aggracan/GAG) can be seen from the graphs. These results and performance are inter-translatable in the EF-on-Edge modelling system and the virtually applicable MCG-PTCC system *in vitro.*

Figure 9 are graphs derived from on an alginate hydrogel based MGC-PTCC model from which, by dissolving the alginate construct with sodium citrate, the neo-tissue islets in cavities and the scattered cells remaining in gel phase are separately harvested and their gene expression profiles are shown. Figure 9 shows the RT-PCR results. From these results, the cells from cell-aggregates/cell-islets expressed higher amount of all genes than the monodispersed cells in the gel bulk. Figures 13 and 19 are both RT-PCR results and Figure 16 shows biochemistry assay results. They together show statistically that all cells grow better under PTCC.

## Example 14: Kinetic profiling

Figure 6D is a graph showing determination of agarose content inside the cell-laden constructs along with passage of *in vitro* culture time of a PTCC construct and a control sample without cavities. The kinetics of agarose weight change is quantified with relative agarose remaining percentage until 32 days. From the graph it can be seen that the three-dimensional network experiences a gradual disentanglement under squeezing pressure by expansion of neo-tissue islets, given the physical crosslinking based construction in agarose gel, which results in removal of agarose via linear molecular release.

Figure 8 is a graph showing kinetics of cavity creation quantified with gelatin release percentage over 6 days. The graph shows that about 75% of the gelatin is degraded in 1 day, and about 85% of the gelatin is degraded after 6 days. Within the graph are inserts of optical microscopic images at time of 0, 8 and 24 hours respectively. The images show the disappearance of red luminance of gelatin spheres (pre-labeled with Rhodamine B fluorescent dye) over time.

## Example 15: Formation of scaffold for tissue engineering

The inventors have demonstrated the use of phase transfer cell culture (PTCC) method for self-forming tissue generation. To distinguish the cell aggregates from individual cells in the gel-bulk, alginate was adopted as the kind of hydrogel for fabricating micro-cavitary constructs.

In the experiments, a modified alginate recovered chondrocytes (ARC) method was used. Chondrocytes were resuspended in 1.5 % (weight/volume) low-viscosity alginate solution (Sigma-Aldrich, St. Louis, MO) at 10 million cells/mL with gelatin microspheres prepared as the mentioned above. Alginate sheets were formed by dispensing the alginate-cell-sphere suspension (about 1.2 to about 1.5 ml) into a substrate of 15 % (weight/volume) gelatin solution with 102 mM calcium chloride. After storage in 4 °C for 4 min, an additional 102 mM calcium chloride solution was added on top of the sheets for solidifying the gel. After storage in 4 °C for 4 min, the sheets could be peeled off and cut to smaller pieces. The pieces were then cultivated in culture medium in cell culture plates. The cultures were maintained with media changes every 3 days for up to 35 days.

While this process was ongoing with "phase transfer cell culture" (PTCC) strategy and cell aggregates appearing, the alginate pieces were collected and dissolved in a buffer at pH 6.8 containing 55 mM sodium citrate and 0.15 M sodium chloride, for 15 min at room temperature. The alginate gel parts were dissolved leaving the scaffolds which could be sustained by the cells with their cell-associated matrix. This kind of scaffold has been named by the inventors as "living scaffold", as opposed to that obtained by the break up of plain non-cavitary hydrogels (serving as Control). This novel scaffold could be further cultivated in culture medium for another 35 days to be more similar to native cartilage chip.

Figure 10 is a schematic illustration of scaffold for tissue engineering formation. Stage (i) to (vii) have previously been explained in Figure 1, i.e. in stage (i), a composition comprising a degradable hydrogel precursor 101', a degradable particle 102, and a living cell 103 is provided. In this embodiment, living cells 103 and degradable particles 102 are suspended together in a degradable hydrogel precursor 101' in an aqueous medium. Subsequently, the composition can be set or solidified to form a degradable hydrogel 101, wherein the degradable hydrogel can encapsulate the degradable particles 102 and the living cells 103, as shown in stage (ii). The hydrogel matrix comprising degradable hydrogel 101, degradable particles 102 and living cells 103 are subjected to certain conditions, which can cause the degradable particles 102 to degrade, forming degraded products which can permeate through the hydrogel matrix, as represented by the outward pointing arrows in stage (iv). Consequently, cavities 105 can be formed in the area originally occupied by the degradable particles 102 in the hydrogel matrix as shown in stage (v). In stage (vi), living cells 103 dispersed in the degradable hyrogel 101 can proliferate in the hydrogel. In addition, the living cells 103 can migrate or move towards the cavities. In stage (vii), the living cells 103 can continue to grow and form neo-tissues, which can eventually fill up the cavities thereby forming islets, which can be spherical. In stage (iii), the islets can fuse together, forming a network of interconnecting tissues. In stage (viii), the hydrogel is degraded from the hydrogel matrix comprising the generated fused tissues from stage (iii). Subsequently, a cartilage-like tissue can be formed from either of two pathways, i.e. (a) by culturing without or (b) with adding cells exogenously.

Figure 11 is a schematic illustration of a method to form a scaffold for tissue engineering by adding extra cells. As shown in the figure, the tissue is cultured after gel detachment in plate for 7 days to enhance stability of the tissue. Subsequently, the cultured tissue is transferred to an insert, followed by centrifuge to remove surrounding medium. Cells are then into the tissue, followed by culturing it in the insert system for 7 days, and then transferring the tissue to a normal culture plate for culturing until cartilage-like tissue are obtained.

Figure 12 is a photograph illustration of the formation of scaffold for tissue engineering, as well as comparison of a scaffold for tissue engineering according to an embodiment of the present invention (denoted by (b)), and a native cartilage chip (denoted by (a)), i.e. part of a natural cartilage removed from a bone used in the experiment. The results show that the scaffold obtained from an embodiment of the present invention closely resembles the native cartilage chip obtained from the bone.

Figure 13 are graphs showing an analysis of the expression of relevant cartilaginous markers (i) Collagen Type II, (ii) Aggrecan, (iii) RhoA, (iv) Integrin $\beta$1, (v) COMP, (vi) Sox 9, and (vii) non-cartilaginous marker (Collagen Type I) at transcriptional level using samples from porcine chondrocytes in alginate hydrogel with cavities formed using a method according to the present invention (denoted as "PTCC") and control samples of chondrocytes in conventional alginate hydrogel (denoted as "Control"), as well as both native Cartilage (denoted as "Cartilage") and Chondrocyte Passage 1 (denoted as "Chondrocyte P1") samples by quantitative real-time PCR (n = 3) after 2, 7, 21 and 35 days. The values represent the levels of expression relative to that of the internal control gene (TBP1) and are expressed as means ± standard deviations. PTCC samples showed better performance than Control. (The notation * indicates differences between different constructs at the same culture time are not significant ($p > 0.05$) and the notation ** indicates differences between different constructs at the same culture time are significant ($p < 0.05$).)

Figure 14 shows photographs of results of (a) PTCC scample scaffold for tissue engineering according to an embodiment of the present invention and (b) control sample of chondrocytes in conventional alginate hydrogel after the detachment of alginate hydrogels (indicated by arrows). As shown, after alginate was detached, scaffold sample according to an embodiment of the present invention could still hold its whole shape as an integral construct.

Figure 15 is a graph showing cell proliferation in (a) scaffold for tissue engineering according to an embodiment of the present invention (denoted as "PTCC") and (b) control sample of chondrocytes in conventional alginate hydrogel (denoted as "Control") examined with WST-1 colorimetric assay. As shown, cell viability in the PTCC samples (about 280%) was significant better by about 1.6 times than that in control samples (about 180%).

Figure 16 are graphs showing investigation of the secretion of extracellular matrix (ECM) of glycosaminoglycan (GAG) and total collagen in samples from porcine chondrocytes in alginate hydrogel with cavities formed using a method according to the present invention (denoted as "PTCC") and control samples of chondrocytes in conventional alginate hydrogel (denoted as "Control") as a function of culture time at 2, 7, 21, and 35 days. (i) and (iii) shows graphs of GAG expressed in terms of GAG secretion (ng/cell) and GAG content (mg/mg dry weight) respectively. (ii) and (iv) shows graphs of total collagen expressed in terms of total collagen secretion (ng/cell) and total collagen content (mg/mg dry weight) respectively. Moreover, the secretion in PTCC samples which underwent gel detachment was also examined. As shown, regarding the ECM secretion amount either in GAG or total collagen, PTCC samples performed higher than control over time. (The notation * indicates differences between different constructs at the same culture time are not significant ($p > 0.05$) and the notation ** indicates differences between different constructs at the same culture time are significant ($p < 0.05$).)

Figure 17 are optical microscope images of histological evaluation of samples from porcine chondrocytes in alginate hydrogel with cavities formed using a method according to the present invention (denoted as "PTCC") cultured for 35 days in contrast with control samples of chondrocytes in conventional alginate hydrogel (denoted as "Control"). EF-side refers to the edge flourish side which is from the edge of the hydrogel and not from the hydrogel cavity.

Figure 17A shows application of hematoxylin and eosin (H&E) stain to survey the histological evolution.

Figure 17B shows application of Safranin-O stain to reveal positive glycosaminoglycan (GAG) production in red.

Figure 17C shows Live/Dead staining (mainly stained by green fluorescent calcein) was applied to test chondrocyte viability in the hydrogel samples. As shown by the areas highlighted with dashed circles in the images, the process of cell sprouting with subsequent cell colony filling-up and tissue fusing was reproduced in PTCC samples, in contrast to the results of the Control samples, in which such cell colonies and tissue fusing were not observed.

Figure 18 shows optical microscope images of PTCC tissue development, which underwent the detachment of alginate hydrogels and subsequently cultured either (i) with or (ii) without extra addition of additional cells. As shown, more and more cell colonies appeared and fused together within the PTCC tissues (a few such areas are exemplarily highlighted with a circle) as a function of culture time, both in the above two kinds of tissues.

Figure 19 are graphs showing analysis of the expression of relevant cartilaginous markers (i) Collagen Type II, (ii) Aggrecan, (iii) COMP, (iv) Sox 9, (v) RhoA, (vi) Integrin ($\beta$1) and (vii) non-cartilaginous marker (Collagen Type I) at transcriptional level from porcine chondrocytes that were encapsulated in scaffold according to an embodiment of the present invention with (denoted by "PTCC_w adding") or without (denoted by "PTCC_w/o adding") adding cells, as well as native cartilage (denoted by "Cartilage") and Chondrocyte Passage 1 (denoted by "Chondrocyte P1") samples by quantitative real-time PCR (n = 3) after 2, 7, 21 and 35 days. PTCC(-0) samples point to the samples cultured for 35 days before gel-detachment. The values represent the levels of expression relative to that of the internal control gene (TBP1) and are expressed as means $\pm$ standard deviations. As shown, the living scaffolds performed stable gene expression even after gel detachment. (The notation * indicates differences between different constructs at the same culture time are not significant ($p > 0.05$) and the notation ** indicates differences between different constructs at the same culture time are significant ($p < 0.05$).)

## Claims

**1.** A composition comprising one or more species of living cells, and a mixture of at least one degradable hydrogel and at least one kind of degradable and surface cross-linked particle, wherein the at least one kind of degradable and surface cross-linked particle comprises a material which degrades faster than the at least one degradable hydrogel, wherein the at least one kind of degradable and surface cross-linked particle has a surface comprising additional reactive groups to bind the one or more species of living cells to its surface, and wherein the one or more species of living cells bound to the surface of the at least one kind of degradable and surface cross-linked particle are comprised in the at least one degradable hydrogel, and wherein the at least one degradable hydrogel of the mixture is formable to a hydrogel matrix.

**2.** The composition of claim 1, wherein

(1) the at least one degradable hydrogel or the at least one kind of degradable and surface cross-linked particle or the at least one degradable hydrogel and the at least one kind of degradable and surface cross-linked particle are biodegradable; and/or
(2) the at least one kind of degradable and surface cross-linked particle is a spherical particle; or a degradable microparticle or a degradable spherical microparticle; and/or
(3) the at least one kind of degradable and surface cross-linked particle has a maximal dimension of between 100 μm to 5 mm or has a maximal dimension of between 200 μm to 600 μm.

**3.** The composition of any one of the preceding claims, wherein the at least one degradable hydrogel is selected from the group consisting of hydrogels made from natural polymers, hydrogels made from synthetic polymers, and their combination thereof, and
wherein

(1) the natural polymer is optionally selected from the group consisting of polysaccharides, glycosaminoglycans, and proteins, and
wherein the polysaccharide is optionally selected from the group consisting of alginate, agarose, chitosan, dextran, starch, and gellan gum; or
(2) the synthetic polymer is optionally selected from the group consisting of hydrogels made from a hydrophilic monomer, hydrogels made from a hydrophilic polymer, hydrogels made from a hydrophilic copolymer, and combinations thereof, and

wherein

(i) the hydrophilic monomer is optionally selected from the group consisting of 2-hydroxyethyl methacrylate, 2-hydroxyethyl acrylate, 2-hydroxyethyl methacrylamide, 2-hydroxyethyl acrylamide, N-2-hydroxyethyl vinyl carbamate, 2-hydroxyethyl vinyl carbonate, 2-hydroxypropyl methacrylate, hydroxyhexyl methacrylate, hydroxyoctyl methacrylate, acrylic acid, methacrylic acid, itaconic acid, fumaric acid, crotonic acid, maleic acid, monomethyl maleate ester, monoethyl maleate ester, monomethyl fumarate ester, monoethyl fumarate ester, (meth)acrylamide, crotonic amide, cinnamic amide, maleic diamide, fumaric diamide, methanethiole, ethanethiol, 1-propanethiol, butanethiol, tert-butyl mercaptan, pentanethiols, p-styrenesulfonic acid, vinylsulfonic acid, p-a-methylstyrenesulfonic acid, isoprene sulfonide and salts thereof, or
(ii) the hydrophilic polymer is optionally selected from group consisting of polymers and oligomers of glycolide, lactide, polylactic acid, polyesters of a-hydroxy acids, including lactic acid and glycolic acid, such as the poly(a-hydroxy) acids including polyglycolic acid, poly-DL-lactic, poly-L-lactic acid, and terpolymers of DL-lactide and glycolide, e-caprolactone and e-caprolactone copolymerized with polyesters, polylactones and polycaprolactones including poly(e-caprolactone), poly(8-valerolactone) and poly (gamma-butyrolactone); polyanhydrides, polyorthoesters, other hydroxy acids, polydioxanone, collagen-hydroxyethylmethacrylate (HEMA), poly(hydroxylethyl methacrylate) (PHEMA), and other biologically degradable polymers that are non-toxic or are present as metabolites in the body.

**4.** The composition of any one of the preceding claims, wherein the at least one kind of degradable and surface cross-linked particle comprises a degradable material which (i) degrades at body temperature; and/or (ii) is selected from the group consisting of hydrocarbon, hydrogel and organic compound, wherein the hydrogel is optionally surface cross-linked gelatine, and/or is chemically functionalized by subjecting a degradable particle to a cross-linking agent.

**5.** The composition of any one of the preceding claims, wherein the weight ratio of degradable hydrogel and degradable and surface cross-linked particle in the composition is between 0.01:1 to 1:1.

**6.** The composition of any one of the preceding claims, wherein the living cells are eukaryotic cells or prokaryotic cells or archaea, and wherein

(1) the eukaryotic cells are optionally anchorage dependent cells, and wherein the anchorage dependent cells are optionally selected from the group consisting of osteogenic cells, fibroblasts, epidermal cells, adipocytes, neural cells, endothelial cells, epithelial cells, keratinocytes, hepatocytes, myocytes, cells from joint ligaments, and cells from the nucleus pulposis; or
(2) the eukaryotic cells are optionally non-anchorage dependent cells, and wherein the non-anchorage dependent cells are optionally selected from the group consisting of chondrocytes, embryonic stem cells, adult stem cells, endodermal lineage cells, and carcinoma cells used for regenerative medicine.

**7.** The composition of any one of the preceding claims, wherein the at least one degradable hydrogel and/or the at least one kind of degradable and surface cross-linked particle further comprises at least one kind of biologically active molecule, and,
wherein the at least one kind of biologically active molecule is optionally selected from the group consisting of growth factor, cytokine, antibiotic, antiinflammatory agent, analgesic, and a cell attachment molecule.

**8.** The composition of any one of the preceding claims, wherein the composition is an injectable composition and/or is formed into a scaffold for tissue engineering.

**9.** A method of manufacturing a composition of any one of claims 1-8, wherein the method comprises:

• mixing one or more species of living cells, at least one degradable hydrogel and at least one kind of degradable and surface cross-linked particle to form a hydrogel comprising the one or more species of living cells and the at least one kind of degradable and surface cross-linked particle, wherein the at least one kind of degradable and surface cross-linked particle comprises a material which degrades faster than the at least one degradable hydrogel, wherein the at least one kind of degradable and surface cross-linked particle has a surface comprising additional reactive groups to bind the one or more species of living cells to its surface, and wherein the one or more species of living cells bound to the surface of the at least one kind of degradable and surface cross-linked particle are comprised in the at least one degradable hydrogel, and wherein the at least one degradable hydrogel of the mixture is formable to a hydrogel matrix.

**10.** The method of claim 9, wherein the composition is adapted to be deliverable to a defect site in an animal body, and wherein

(1) the composition is optionally adapted to be deliverable to a defect site in an animal body by injection; and/or
(2) the composition is optionally adapted to induce tissue generation.

**11.** The method of claim 9 or 10, wherein the at least one degradable hydrogel and the at least one kind of degradable and surface cross-linked particle in the composition are mixed together in a weight ratio of between 0.01:1 to 1:1.

**12.** The method of any one of claims 9 to 11, wherein a degradable particle used for the manufacture of a degradable and surface cross-linked particle is manufactured by a double-emulsion method, and
wherein the degradable particle formed using the double-emulsion method is optionally subjected to a cross-linking agent to obtain a degradable and surface cross-linked particle.

**13.** Use of a composition of any one of claims 1-8 or the composition prepared according to any one of claims 9-12 for manufacturing a scaffold for tissue engineering, comprising:

- providing a mixture comprising the composition to form a hydrogel comprising the at least one kind of degradable and surface cross-linked particle and the one or more species of living cells, wherein the at least one kind of degradable and surface cross-linked particle comprises a material which degrades faster than the at least one degradable hydrogel,
- incubating the mixture under conditions which allow proliferation of the one or more species of living cells and degradation of the at least one degradable and surface cross-linked particle in the hydrogel to allow the one or more species of living cells to proliferate;
- degrading the at least one degradable hydrogel of the incubated mixture to obtain a scaffold.

**14.** The use of claim 13, further comprising

(1) further incubation of the scaffold under conditions which allow proliferation of the one ore more species of living cells, and/or
(2) adding one or more species of living cells after incubating the mixture,

wherein the one or more species of living cells added after incubating the mixture comprises the same or different species of cells than the cells already mixed into the mixture together with the at least one degradable hydrogel and the at least one kind of degradable and surface cross-linked particle.

## Patentansprüche

1. Eine Zusammensetzung, umfassend eine oder mehrere Arten an lebenden Zellen und ein Gemisch von mindestens einem abbaubaren Hydrogel und mindestens einer Art eines abbaubaren und an der Oberfläche vernetzten Teilchens, wobei die mindestens eine Art eines abbaubaren und an der Oberfläche vernetzten Teilchens ein Material umfasst, welches sich schneller abbaut als das mindestens eine abbaubare Hydrogel, wobei die mindestens eine Art eines abbaubaren und an der Oberfläche vernetzten Teilchens eine Oberfläche besitzt, welche zusätzliche reaktive Reste umfasst, um die eine oder mehreren Arten an lebenden Zellen an ihre Oberfläche zu binden, und wobei die eine oder mehreren Arten an lebenden Zellen, welche an die Oberfläche der mindestens einen Art eines abbaubaren und an der Oberfläche vernetzten Teilchens gebunden ist in dem mindestens einen abbaubaren Hydrogel enthalten ist und wobei das mindestens eine abbaubare Hydrogel des Gemischs zu einer Hydrogelmatrix formbar ist.

2. Die Zusammensetzung gemäß Anspruch 1, wobei

(1) das mindestens eine abbaubare Hydrogel oder die mindestens eine Art eines abbaubaren und an der Oberfläche vernetzten Teilchens oder das mindestens eine abbaubare Hydrogel und die mindestens eine Art eines abbaubaren und an der Oberfläche vernetzten Teilchens bio-abbaubar sind; und/oder
(2) die mindestens eine Art eines abbaubaren und an der Oberfläche vernetzten Teilchens ein kugelförmiges Teilchen ist; oder ein abbaubares Kleinstteilchen ist oder ein abbaubares kugelförmiges Kleinstteilchen ist; und/oder
(3) die mindestens eine Art eines abbaubaren und an der Oberfläche vernetzten Teilchens eine maximale Größe zwischen 100 $\mu$m bis 5 mm aufweist oder eine maximale Größe zwischen 200 $\mu$m bis 600 $\mu$m aufweist.

3. Die Zusammensetzung gemäß einem der vorstehenden Ansprüche, wobei das mindestens eine abbaubare Hydrogel ausgewählt ist aus der Gruppe bestehend aus Hydrogelen, welche hergestellt sind aus natürlichen Polymeren, Hydrogelen, welche hergestellt sind aus synthetischen Polymeren und die Kombination davon und wobei

(1) das natürliche Polymer gegebenenfalls ausgewählt ist aus der Gruppe bestehend aus Polysacchariden, Glykosaminoglykanen und Proteinen und
wobei das Polysaccharid gegebenenfalls ausgewählt ist aus der Gruppe bestehend aus Alginat, Agarose, Chitosan, Dextran, Stärke und Gellan; oder
(2) das synthetische Polymer gegebenenfalls ausgewählt ist aus der Gruppe bestehend aus Hydrogelen, welche hergestellt sind aus einem hydrophilen Monomer, Hydrogelen, welche hergestellt sind aus einem hydrophilen Polymer, Hydrogelen, welche hergestellt sind aus einem hydrophilen Copolymer und Kombinationen davon, und

wobei

(i) das hydrophile Monomer gegebenenfalls ausgewählt ist aus der Gruppe bestehend aus 2-Hydroxyethylmethacrylat, 2-Hydroxyethylacrylat, 2-Hydroxyethylmethacrylamid, 2-Hydroxyethylacrylamid, N-2-Hydroxyethylvinylcarbamat, 2-Hydroxyethylvinylcarbonat, 2-Hydroxypropylmethacrylat, Hydroxyhexylmethacrylat, Hydroxyoctylmethycrylat, Acrylsäure, Methacrylsäure, Itaconsäure, Fumarsäure, Krotonsäure, Maleinsäure, Monomethylmaleatester, Monoethylmaleatester, Moomethylfumaratester, Monoethylfumaratester, (Meth)acrylamid, Krotonamid, Zimtsäureamid, Maleinsäurediamid, Fumarsäurediamid, Methanthiol, Ethanthiol, 1-Propanthiol, Butanthiol, tert-Butylmercaptan, Pentanthiol, p-Styrolsulfonsäure, Vinylsulfonsäure, p-a-Methylstyrolsulfonsäure, Isoprensulfonsäure und Salze davon, oder
(ii) das hydrophilie Polymer gegebenenfalls ausgewählt ist aus der Gruppe bestehend aus Polymeren und Oligomeren von Glykolsäure, Lactid, Polymilchsäuren, Polyestern von a-Hydroxysäuren, einschließlich Milchsäure und Glykolsäure, wie zum Beispiel die Poly(a-Hydroxy)säuren, einschließlich Polyhydroxyessigsäure, Poly-DL-Milchsäure, Poly-L-Milchsäure, und Terpolymere von DL-Lactid und Glykolsäure, e-Caprolacton und e-Caprolacton, welches mit Polyestern, Polylactonen und Polycaprolactonen, einschließlich Poly(e-Caprolacton), Poly(8-Valerolacton) und Poly(gamma-Butyrolacton) copolymerisiert ist; Polyanhydriden, Polyorthoestern, anderen Hydroxycarbonsäuren, Poly-p-dioxanon, Kollagenhydroxyethylmethacrylaten (HEMA), Poly(hydroxylethylmethacrylat) (PHEMA) und anderen biologisch abbaubaren Polymeren, welche nicht toxisch sind oder als Stoffwechselprodukt im Körper vorkommen.

**4.** Die Zusammensetzung gemäß einem der vorstehenden Ansprüche, wobei die mindestens eine Art eines abbaubaren und an der Oberfläche vernetzten Teilchens ein abbaubares Material umfasst, welches

(i) sich bei Körpertemperatur abbaut; und/oder
(ii) ausgewählt ist aus der Gruppe bestehend aus Kohlenwasserstoffen, Hydrogel und organischen Verbindungen, wobei das Hydrogel gegebenenfalls eine oberflächenvernetzte Gelatine ist und/oder chemisch funktionalisiert ist, durch Aussetzen des abbaubaren Teilens an einem Vernetzungsmittel.

**5.** Die Zusammensetzung gemäß einem der vorstehenden Ansprüche, wobei das Gewichtsverhältnis von abbaubarem Hydrogel und abbaubarem an der Oberfläche vernetzten Teilchen in der Zusammensetzung 0,01:1 bis 1:1 beträgt.

**6.** Die Zusammensetzung gemäß einem der vorstehenden Ansprüche, wobei die lebenden Zellen eukaryotische Zellen oder prokaryotische Zellen oder Archaeen sind, und wobei

(1) die eukaryotischen Zellen gegebenenfalls verankerungsabhängige Zellen sind und wobei die verankerungsabhängigen Zellen gegebenenfalls ausgewählt sind aus der Gruppe bestehend aus osteogenen Zellen, Fibroblasten, Epidermalzellen, Adipozyten, Nervenzellen, Endothelzellen, Epithelzellen, Keratinozyten, Hepatozyten, Myozyten, Zellen aus Gelenkbändern und Zellen aus dem Nucleus Pulposus; oder
(2) die eukaryotischen Zellen gegebenenfalls keine verankerungsabhängigen Zellen sind und wobei die nicht verankerungsabhängigen Zellen gegebenenfalls ausgewählt sind aus der Gruppe bestehend aus Chondrozyten, embryonischen Stammzellen, adulten Stammzellen, endodermale Stammzellen, und Krebszellen, welche für regenerative Medizin verwendet werden.

**7.** Die Zusammensetzung gemäß einem der vorherigen Ansprüche, wobei das mindestens eine abbaubare Hydrogel und/oder die mindestens eine Art eines abbaubaren und an der Oberfläche vernetzten Teilchens ferner mindestens eine Art eines biologisch aktiven Moleküls umfassen, und wobei die mindestens eine Art an biologisch aktivem Molekül gegebenenfalls ausgewählt ist aus der Gruppe bestehend aus Wachstumsfaktor, Zytokin, Antibiotika, Entzündungshemmer, Schmerzmittel und Zelladhäsionsmolekül.

**8.** Die Zusammensetzung gemäß einem der vorherigen Ansprüche, wobei die Zusammensetzung eine injizierbare Zusammensetzung ist und/oder in ein Gerüst für Gewebezüchtung geformt wird.

**9.** Ein Verfahren zur Herstellung einer Zusammensetzung gemäß einem der Ansprüche 1 bis 8, wobei das Verfahren umfasst:

• Mischen der einen oder mehreren Arten an lebenden Zellen, des mindestens einen abbaubaren Hydrogels und der mindestens einen Art eines abbaubaren und an der Oberfläche vernetzten Teilchens, um ein Hydrogel zu bilden, welches die mindestens eine oder mehreren Arten an lebenden Zellen und die mindestens eine Art eines abbaubaren und an der Oberfläche vernetzten Teilchens umfasst, wobei die mindestens eine Art eines abbaubaren und an der Oberfläche vernetzten Teilchens ein Material umfasst, welches sich schneller abbaut als das mindestens eine abbaubare Hydrogel, wobei die mindestens eine Art eines abbaubaren und an der Oberfläche vernetzten Teilchens eine Oberfläche besitzt, welche zusätzliche reaktive Reste umfasst, um die eine oder mehreren Arten an lebenden Zellen an ihre Oberfläche zu binden, und wobei die eine oder mehreren Arten an lebenden Zellen, welche an die Oberfläche der mindestens einen Art eines abbaubaren und an der Oberfläche vernetzten Teilchens gebunden sind in dem mindestens einen abbaubaren Hydrogel enthalten sind und wobei das mindestens eine abbaubare Hydrogel des Gemischs zu einer Hydrogelmatrix formbar ist.

**10.** Das Verfahren gemäß Anspruch 9, wobei die Zusammensetzung so angepasst ist, dass sie einer Schadstelle in einem Tierkörper verabreicht werden kann, und
wobei

(1) die Zusammensetzung gegebenenfalls so angepasst ist, dass sie durch Injektion einer Schadstelle in einem Tierkörper verabreicht werden kann; und/oder
(2) die Zusammensetzung gegebenenfalls angepasst ist, um Zellwachstum einzuleiten.

**11.** Das Verfahren gemäß Anspruch 9 oder 10, wobei das mindestens eine abbaubare Hydrogel und die mindestens eine Art eines abbaubaren und an der Oberfläche vernetzten Teilchens in der Zusammensetzung in einem Gewichtsverhältnis von 0,01:1 zu 1:1 zusammengemischt werden.

**12.** Das Verfahren gemäß einem der Ansprüche 9 bis 11, wobei ein abbaubares Teilchen, welches zur Herstellung eines abbaubaren und an der Oberfläche vernetzten Teilchens genutzt wird, durch ein Doppel-Emulsionsverfahren hergestellt wird, und
wobei das durch das Doppel-Emulsionsverfahren geformte abbaubare Teilchen gegebenenfalls einem Vernetzer ausgesetzt wird, um ein abbaubares und an der Oberfläche vernetztes Teilchen zu erhalten.

**13.** Verwendung einer Zusammensetzung gemäß einem der Ansprüche 1 bis 8 oder der Zusammensetzung hergestellt gemäß einem der Ansprüche 9 bis 12 zur Herstellung eines Gerüsts für Gewebezüchtung, umfassend:

- Bereitstellen einer Mischung, umfassend die Zusammensetzung, um eine Hydrogel zu formen, umfassend die mindestens eine Art eines abbaubaren und an der Oberfläche vernetzten Teilchens und die eine oder mehreren Arten an lebenden Zellen, wobei die mindestens eine Art eines abbaubaren und an der Oberfläche vernetzten Teilchens ein Material umfasst, welches sich schneller abbaut als das mindestens eine abbaubare Hydrogel,
- Inkubieren der Mischung unter Bedingungen, welche die Zellvermehrung der einen oder mehreren Arten an lebenden Zellen und den Abbau des mindestens einen abbaubaren und an der Oberfläche vernetzten Teilchens in dem Hydrogel ermöglichen, um einen oder mehreren Arten an leben Zellen die Zellvermehrung zu ermöglichen;
- Abbauen des mindestens einen abbaubaren Hydrogels der inkubierten Mischung, um ein Gerüst zu erhalten.

**14.** Die Verwendung gemäß Anspruch 13, ferner umfassend

(1) weiteres Inkubieren des Gerüsts unter Bedingungen, welche die Zellvermehrung der einen oder mehreren Arten an lebenden Zellen ermöglichen, und/oder
(2) Zugeben einer oder mehreren Arten an lebenden Zellen nach Inkubieren der Mischung, wobei die eine oder mehreren Arten an lebenden Zellen, welche nach dem Inkubieren der Mischung zugegeben werden, die gleichen oder verschiedene Zellen umfassen, als die Zellen, die bereits in die Mischung zusammen mit dem mindestens einen abbaubaren Hydrogel und der mindesten einen Art eines abbaubaren und an der Oberflächen vernetzten Teilchens gemischt wurden.

## Revendications

**1.** Composition comprenant une ou plusieurs espèces de cellules vivantes, et un mélange d'au moins un hydrogel dégradable et d'au moins un type de particule dégradable et réticulée en surface, dans laquelle l'au moins un type de particule dégradable et réticulée en surface comprend un matériau qui se dégrade plus rapidement que l'au moins un hydrogel dégradable, dans laquelle l'au moins un type de particule dégradable et réticulée en surface a une surface comprenant des groupes réactifs additionnels pour lier la ou les espèces de cellules vivantes à sa surface, et dans laquelle la ou les espèces de cellules vivantes liées à la surface de l'au moins un type de particule dégradable et réticulée en surface sont contenues dans l'au moins un hydrogel dégradable, et dans laquelle l'au moins un hydrogel dégradable du mélange peut être formé en une matrice d'hydrogel.

**2.** Composition selon la revendication 1, dans laquelle

(1) l'au moins un hydrogel dégradable ou l'au moins un type de particule dégradable et réticulée en surface ou l'au moins un hydrogel dégradable et l'au moins un type de particule dégradable et réticulée en surface sont biodégradables ; et/ou
(2) l'au moins un type de particule dégradable et réticulée en surface est une particule sphérique ; ou une microparticule dégradable ou une microparticule sphérique dégradable ; et/ou
(3) l'au moins un type de particule dégradable et réticulée en surface a une dimension maximale entre 100 $\mu$m et 5 mm ou a une dimension maximale entre 200 $\mu$m et 600 $\mu$m.

**3.** Composition selon l'une quelconque des revendications précédentes, dans laquelle l'au moins un hydrogel dégradable est choisi dans le groupe constitué des hydrogels fabriqués à partir de polymères naturels, des hydrogels fabriqués à partir de polymères synthétiques, et de leurs combinaisons, et dans laquelle

(1) le polymère naturel est facultativement choisi dans le groupe constitué des polysaccharides, des glycosaminoglycanes et des protéines, et

dans laquelle le polysaccharide est facultativement choisi dans le groupe constitué de l'alginate, de l'agarose, du chitosane, du dextrane, de l'amidon et de la gomme gellane ; ou
(2) le polymère synthétique est facultativement choisi dans le groupe constitué des hydrogels fabriqués à partir d'un monomère hydrophile, des hydrogels fabriqués à partir d'un polymère hydrophile, des hydrogels fabriqués à partir d'un copolymère hydrophile, et de leurs combinaisons, et

dans laquelle

(i) le monomère hydrophile est facultativement choisi dans le groupe constitué du méthacrylate de 2-hydroxyéthyle, de l'acrylate de 2-hydroxyéthyle, du méthacrylamide de 2-hydroxyéthyle, de l'acrylamide de 2-hydroxyéthyle, du carbamate de N-2-hydroxyéthylvinyle, du carbonate de 2-hydroxyéthylvinyle, du méthacrylate de 2-hydroxypropyle, du méthacrylate d'hydroxyhexyle, du méthacrylate d'hydroxyoctyle, de l'acide acrylique, de l'acide méthacrylique, de l'acide itaconique, de l'acide fumarique, de l'acide crotonique, de l'acide maléique, de l'ester de maléate de monométhyle, de l'ester de maléate de monoéthyle, de l'ester de fumarate de monométhyle, de l'ester de fumarate de monoéthyle, du (méth)acrylamide, de l'amide crotonique, de l'amide cinnamique, du diamide maléique, du diamide fumarique, du méthanethiole, de l'éthanethiol, du 1-propanethiol, du butanethiol, du tert-butyl-mercaptan, des pentanethiols, de l'acide p-styrènesulfonique, de l'acide vinylsulfonique, de l'acide p-a-méthylstyrènesulfonique, de l'isoprène sulfonide et des sels de ceux-ci, ou
(ii) le polymère hydrophile est facultativement choisi dans le groupe constitué des polymères et oligomères de glycolide, de lactide, d'acide polylactique, de polyesters d'a-hydroxy-acides, comprenant l'acide lactique et l'acide glycolique, tels que les poly(a-hydroxy)acides comprenant l'acide polyglycolique, l'acide poly-DL-lactique, poly-L-lactique, et des terpolymères de DL-lactide et de glycolide, d'e-caprolactone et d'e-caprolactone copolymérisée avec des polyesters, de polylactones et de polycaprolactones comprenant la poly(e-caprolactone), la poly(8-valérolactone) et la poly(gamma-butyrolactone) ; des polyanhydrides, des polyorthoesters, d'autres hydroxy-acides, de la polydioxanone, du collagène-hydroxyéthylméthacrylate (HEMA), du poly(hydroxyléthylméthacrylate) (PHEMA), et d'autres polymères biologiquement dégradables qui ne sont pas toxiques ou qui sont présents sous forme de métabolites dans l'organisme.

**4.** Composition selon l'une quelconque des revendications précédentes, dans laquelle l'au moins un type de particule dégradable et réticulée en surface comprend un matériau dégradable qui (i) se dégrade à la température corporelle ; et/ou (ii) est choisi dans le groupe constitué d'un hydrocarbure, d'un hydrogel et d'un composé organique, dans laquelle l'hydrogel est facultativement de la gélatine réticulée en surface, et/ou est chimiquement fonctionnalisé en soumettant une particule dégradable à un agent de réticulation.

**5.** Composition selon l'une quelconque des revendications précédentes, dans laquelle le rapport pondéral de l'hydrogel dégradable et de la particule dégradable et réticulée en surface dans la composition est entre 0,01/1 et 1/1.

**6.** Composition selon l'une quelconque des revendications précédentes, dans laquelle les cellules vivantes sont des cellules eucaryotes ou des cellules procaryotes ou des archéobactéries, et
dans laquelle

(1) les cellules eucaryotes sont facultativement des cellules dépendantes de l'ancrage, et dans laquelle les cellules dépendantes de l'ancrage sont facultativement choisies dans le groupe constitué des cellules ostéogéniques, des fibroblastes, des cellules de l'épiderme, des adipocytes, des cellules neurales, des cellules endothéliales, des cellules épithéliales, des kératinocytes, des hépatocytes, des myocytes, des cellules des ligaments articulaires et des cellules du noyau pulpeux ; ou
(2) les cellules eucaryotes sont facultativement des cellules indépendantes de l'ancrage, et

dans laquelle les cellules indépendantes de l'ancrage sont facultativement choisies dans le groupe constitué des chondrocytes, des cellules souches embryonnaires, des cellules souches adultes, des cellules de la lignée endodermique et des cellules de carcinome utilisées pour la médecine régénérative.

**7.** Composition selon l'une quelconque des revendications précédentes, dans laquelle l'au moins un hydrogel dégradable et/ou l'au moins un type de particule dégradable et réticulée en surface comprend en outre au moins un type de molécule biologiquement active, et,
dans laquelle l'au moins un type de molécule biologiquement active est facultativement choisi dans le groupe constitué d'un facteur de croissance, d'une cytokine, d'un antibiotique, d'un agent anti-inflammatoire, d'un analgésique et d'une molécule d'adhésion cellulaire.

**8.** Composition selon l'une quelconque des revendications précédentes, dans laquelle la composition est une composition injectable et/ou est formée en échafaudage de génie tissulaire.

**9.** Procédé de fabrication d'une composition selon l'une quelconque des revendications 1 à 8, dans lequel le procédé comprend :

• le mélange d'une ou plusieurs espèces de cellules vivantes, d'au moins un hydrogel dégradable et d'au moins un type de particule dégradable et réticulée en surface pour former un hydrogel comprenant la ou les espèces de cellules vivantes et l'au moins un type de particule dégradable et réticulée en surface, dans lequel l'au moins un type de particule dégradable et réticulée en surface comprend un matériau qui se dégrade plus rapidement que l'au moins un hydrogel dégradable, dans lequel l'au moins un type de particule dégradable et réticulée en surface a une surface comprenant des groupes réactifs additionnels pour lier la ou les espèces de cellules vivantes à sa surface, et dans lequel la ou les espèces de cellules vivantes liées à la surface de l'au moins un type de particule dégradable et réticulée en surface sont contenues dans l'au moins un hydrogel dégradable, et dans lequel l'au moins un hydrogel dégradable du mélange peut être formé en une matrice d'hydrogel.

**10.** Procédé selon la revendication 9, dans lequel la composition est adaptée pour pouvoir être administrée à un site présentant un défaut du corps d'un animal, et
dans lequel

(1) la composition est facultativement adaptée pour pouvoir être administrée à un site présentant un défaut du corps d'un animal par injection ; et/ou
(2) la composition est facultativement adaptée pour induire la génération des tissus.

**11.** Procédé selon la revendication 9 ou 10, dans lequel l'au moins un hydrogel dégradable et l'au moins un type de particule dégradable et réticulée en surface dans la composition sont mélangés l'un à l'autre dans un rapport pondéral entre 0,01/1 à 1/1.

**12.** Procédé selon l'une quelconque des revendications 9 à 11, dans lequel une particule dégradable utilisée pour la fabrication d'une particule dégradable et réticulée en surface est fabriquée par un procédé de double émulsion, et dans lequel la particule dégradable formée à l'aide du procédé de double émulsion est facultativement soumise à un agent de réticulation pour obtenir une particule dégradable et réticulée en surface.

**13.** Utilisation d'une composition selon l'une quelconque des revendications 1 à 8 ou de la composition préparée selon l'une quelconque des revendications 9 à 12 pour la fabrication d'un échafaudage de génie tissulaire, comprenant :

▪ la fourniture d'un mélange comprenant la composition pour former un hydrogel comprenant l'au moins un type de particule dégradable et réticulée en surface et la ou les espèces de cellules vivantes, dans laquelle l'au moins un type de particule dégradable et réticulée en surface comprend un matériau qui se dégrade plus rapidement que l'au moins un hydrogel dégradable,
▪ l'incubation du mélange dans des conditions qui permettent la prolifération de la ou des espèces de cellules vivantes et la dégradation de l'au moins une particule dégradable et réticulée en surface dans l'hydrogel pour permettre à la ou aux espèces de cellules vivantes de proliférer ;
▪ la dégradation de l'au moins un hydrogel dégradable du mélange incubé pour obtenir un échafaudage.

**14.** Utilisation selon la revendication 13, comprenant en outre

(1) une nouvelle incubation de l'échafaudage dans des conditions qui permettent la prolifération de la ou des espèces de cellules vivantes, et/ou
(2) l'ajout d'une ou plusieurs espèces de cellules vivantes après incubation du mélange, dans laquelle la ou les espèces de cellules vivantes ajoutées après incubation du mélange comprend (comprennent) des espèces de cellules identiques ou différentes des cellules déjà mélangées dans le mélange conjointement avec l'au moins un hydrogel dégradable et l'au moins un type de particule dégradable et réticulée en surface.

FIG. 1A
(i)
101'
102
103
101
102
103
(ii)
200 μm
FIG. 1B
102
104
101
102
103
101
105
103
FIG. 1C
101
102
103
(i)
102
103
105
(ii)
103
105
(iii)
200 μm
105

FIG. 1D

101
103
105
(a)
(b)
(c)
(d)
(i)
(ii)
(iii)
(iv)
(v)

FIG. 2A

D 10
D 14
500 µm

FIG. 2B

FIG. 3

600 - 800 µm
105
5 mm
105
D3
105
105
D 50
400 µm

FIG. 4

(i)
(ii)
(a)
(b)
500 μm

FIG. 5A

(a)
(b)
(i)
(ii)
(iii)
(iv)

**FIG. 5B**

RhoA expression (2 -ΔCT)
0.04
0.02
0.00
D3
D10
D18
D32
(i)
COMP expression (2 -ΔCT)
1.2
0.6
0.0
D3
D10
D18
D32
(ii)
Collagen II expression (2 -ΔCT)
100
50
0
D3
D10
D18
D32
(iii)
Sox9 expression (2 -ΔCT)
0.0018
0.0009
0.0000
D3
D10
D18
D32
(iv)
Integrin β1 expression (2 -ΔCT)
0.08
0.04
0.00
D3
D10
D18
D32
(v)
Surface
Bulk
Aggrecan expression (2 -ΔCT)
4
2
0
D3
D10
D18
D32
(vi)

# FIG. 5C

Surface
Bulk
300
150
0
GAG secretion (mg/g)
**
D10
D18
D32
(i)
240
120
0
Collagen secretion (mg/g)
(ii)

# FIG. 6A

RhoA Normalized Fold Expression
2
1
0
D3
D10
D18
D32
(i)
Sox 9 Normalized Fold Expression
(ii)
PTCC
Control
Aggrecan Normalized Fold Expression
1.6
0.8
0.0
(iii)
Collagen II Normalized Fold Expression
(iv)
**
*

FIG. 6B

GAG secretion (x10⁻⁴ μg/cell)
30
15
0
D3
D7
D12
D18
D24
D32
(i)
PTCC
Control
Collagen secretion (x10⁻⁴ μg/cell)
24
12
0
D7
D12
D18
D24
D32
(ii)
FIG. 6C
(i)
(ii)
(a)
100μm
(b)
50μm

FIG. 6D

Relative Agarose Remaining Percentage (%)
100
90
80
70
60
50
PTCC
Control
D0 D3
D32

FIG. 7

(a)
(b)
(i) D3
(ii) D12
(iii) D40
200 μm

FIG. 8

Gelatin Release Percentage (%)
90
75
60
45
30
15
0
Time (Day)
0
1
4
6
0 hour
8 hours
24 hours
200μm

FIG. 9

RhoA Normalized Fold Expression
1.8
0.9
0.0
D4
D16
D24
D32
(i)
COMP Normalized Fold Expression
9
6
3
0
D4
D16
D24
D32
(ii)
Collagen II Normalized Fold Expression
10
5
0
D4
D16
D24
D32
(iii)
Sox 9 Normalized Fold Expression
20
10
0
D4
D16
D24
D32
(iv)
Aggrecan Normalized Fold Expression
12
6
0
D4
D16
D24
D32
(v)
Mixed
Islet
In-bulk

FIG. 10
(i)
101'
102
103
(ii)
101
102
103
(iii)
(iv)
102
(v)
105
103
(vi)
103
(vii)
(viii)
(a)
(b)
(ix)
(x)
(xi)
(xii)

FIG. 11

(i)
(vi)
(v)
(ii)
(iii)
(iv)

FIG. 12

(b)
(a)

Collagen II Normalized Fold Expression
0
4
8
12
-33
-28
-14
-0

(i)

Aggrecan Normalized Fold Expression
0
3
6
9
12
-33
-28
-14
-0

(ii)

RhoA Normalized Fold Expression
0
2
4
6
-33
-28
-14
-0

(iii)

Integrin β1 Normalized Fold Expression
0
1
2
-33
-28
-14
-0

(iv)

COMP Normalized Fold Expression
0
2
4
6
8
-33
-28
-14
-0

(v)

Sox9 Normalized Fold Expression
0
2
4
6
8
-33
-28
-14
-0

(vi)

FIG. 13 (cont.)

Cartilage
Chondrocyte P1
PTCC(-)
Control
Collagen I Normalized Fold Expression
0
1
2
3
*
**
**
**
-33
-28
-14
-0

(vii)

FIG. 14

1.5 cm
(b)
(a)
0.6 cm
(a)
(b)

FIG. 15

PTCC
Control
PTCC(+0)
3.0
2.5
2.0
1.5
1.0
0.5
Cell Viability (x 100%)
-32 -27 -22 -17 -11 -5 -0 +0
Culture Time (Day)

FIG. 16

Legend:
Cartilage
PTCC(-)
Control
PTCC(+0)
GAG secretion (ng/cell)
-33 -28 -14 -0 +0
(i)
Total Collagen secretion (ng/cell)
-33 -28 -14 -0 +0
(ii)
GAG content (mg / mg dry wt.)
-33 -28 -14 -0 +0
(iii)
Total collagen content (mg / mg dry wt.)
-33 -28 -14 -0 +0
(iv)

**FIG. 17A**

(a) Day18
(b) Day 35
(before alginate detachment)
(c) Day 35
(after alginate detachment)
(d) EF-Side
: PTCC
: Control
200 μm

**FIG. 17B**

(a) Day18

(b) Day 35
(before alginate detachment)

(c) Day 35
(after alginate detachment)

(d) EF-Side

: PTCC

: Control

200 μm

**FIG. 17C**

(a) Day12
(b) Day 24
(c) Day 35
(before alginate detachment)
(d) Day 35
(after alginate detachment)
(e)
: PTCC
: Control
EF-Side
EF-Top
200 μm

FIG. 18

i
ii
Day +29
Day +9
Day +2
Day -14

# FIG. 19

Collagen II Normalized Fold Expression
2
0
-0
+18
+35
*
*
(i)
Aggrecan Normalized Fold Expression
2
0
-0
+18
+35
*
**
(ii)
COMP Normalized Fold Expression
2
0
-0
+18
+35
*
*
(iii)
Sox9 Normalized Fold Expression
6
4
2
0
-0
+18
+35
*
*
(iv)

**FIG. 19 (cont.)**

(v)

(vi)

(vii)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2002150604 A
- WO 10775175 A
- US 61178697 B


### Non-patent literature cited in the description


- **K. MASUDA et al.** *J. Orthop. Res.,* 2003, vol. 21, 139
- **YAPHE et al.** *Anal. Biochem.,* 1965, vol. 13, 143